# EUROPEAN PATENT APPLICATION

(11) **EP 4 477 216 A1**
(43) Date of publication of application: **18.12.2024**
(21) Application number: 23753207.2
(22) Date of filing: 10.02.2023
(51) Int. Cl.: A61K 31/12, A61K 31/704, A61K 31/635, A61K 31/606, A61K 9/06, A61K 47/32, A61K 47/36, A61P 17/16, A61P 29/00, A61P 1/00

(54) **PHARMACEUTICAL COMPOSITION COMPRISING CURCUMIN AND GINSENOSIDE, AND FORMULATION THEREOF**

(30) Priority: 10.02.2022 KR 20220017693
(71) Applicant: CorestemChemon Inc., Seongnam-si, Gyeonggi-do 13486 (KR); Ewha University-Industry Collaboration Foundation, Seoul 03760 (KR)
(72) Inventor: LEE, Tae Yong, Yongin-si Gyeonggi-do 17128 (KR); KIM, Min Sung, Seoul 05771 (KR); YANG, Su Jeong, Seoul 02624 (KR); LEE, Ryung-Ah, Seoul 07034 (KR); KIM, Seong-Eun, Seoul 06279 (KR)
(74) Representative: HGF
(86) International application number: PCT/KR2023/001992
(87) International publication number: WO 2023/153861

(57) **Abstract**

The present invention relates to a pharmaceutical composition comprising curcumin and ginsenoside, and a formulation comprising same. Using curcumin and ginsenoids according to the present invention in combination with butyric acid and sucralfate restores cell growth, mobility, and endothelial cell tube-forming ability reduced by irradiation, and inhibits an inflammatory response induced in macrophages by LPS treatment. In addition, it was confirmed that, when an irradiated mouse is topically treated with the formulation according to the present invention comprising the compounds, intestinal epithelial tissue damaged by irradiation is restored and the expression of inflammatory cytokines in the intestinal tissue is suppressed. Furthermore, a mixture of a ginseng extract and a turmeric extract also restored the intestinal epithelial tissue damaged by irradiation. Therefore, the pharmaceutical composition comprising curcumin, ginsenoid, butyric acid, and sucralfate, or the pharmaceutical composition comprising a mixture of a ginseng extract and a turmeric extract, and the formulation according to the present invention comprising same can be utilized for treating radiation proctitis.

## Description

### TECHNICAL FIELD

The present invention relates to a pharmaceutical composition comprising curcumin and ginsenoside, and a formulation comprising the same.

### BACKGROUND ART

Radiotherapy is one of the most widely used cancer treatments, but is known to have serious side effects. Radiation proctitis, which is characterized by rectal damage, is a major complication of radiotherapy for pelvic cancer, including bladder cancer, cervical cancer, and prostate cancer. Approximately 30% of patients who received pelvic radiotherapy suffer from radiation proctitis, and this side effect reduces the quality of life of patients. Currently, anti-inflammatory or antioxidant agents and formalin are used as therapeutic agents, but their effects are insignificant. Therefore, the development of more effective therapeutic agents is needed.

Curcumin and ginsenoside are compounds derived from *Curcuma longa* and *Panax,* respectively, and are known to have both antioxidant and anti-inflammatory properties. In particular, curcumin has been reported to have effects of reducing the expression of inflammatory cytokines and fibrogenic cytokines in the skin of irradiated mice and restoring skin damage (Okunieff P., et al.(2006) Int J Radiat Oncol Biol Phys 65, 890-898). In addition, ginsenoside Rd has been reported to inhibit apoptosis induced by irradiation in intestinal epithelial cells (Tamura T., et al. (2008) Food chem toxicol 46, 3080-3089).

However, there has been no study yet on whether the combined use of curcumin and ginsenoside can more effectively restore tissue damage caused by radiation.

### DISCLOSURE OF THE INVENTION

### TECHNICAL PROBLEM

Accordingly, the inventors of the present invention conducted research to develop a therapeutic agent for radiation proctitis, an intractable disease, and confirmed that when curcumin and ginsenoside were used in combination, cell damage caused by irradiation was healed. Based on this, the inventors completed the present invention by confirming that a composition containing curcumin and ginsenoside was effective in treatment of radiation proctitis.

### TECHNICAL SOLUTION

To this end, an aspect of the present invention provides a pharmaceutical composition comprising as active ingredients: curcumin having a structure of Chemical Formula 1 or Chemical Formula 2 below or a pharmaceutically acceptable salt thereof; and any one ginsenoside selected from the group consisting of Chemical Formula 3, Chemical Formula 4 and Chemical Formula 5 below or a pharmaceutically acceptable salt thereof:

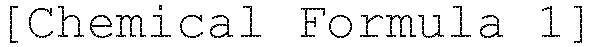

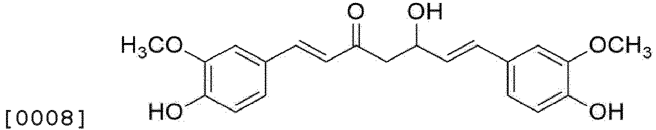

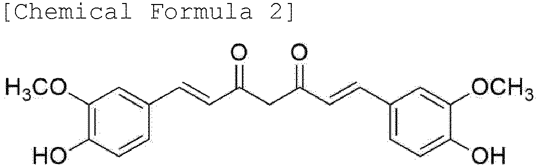

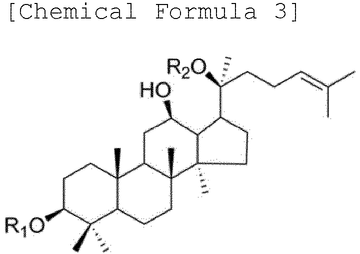

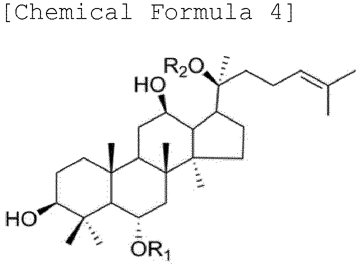

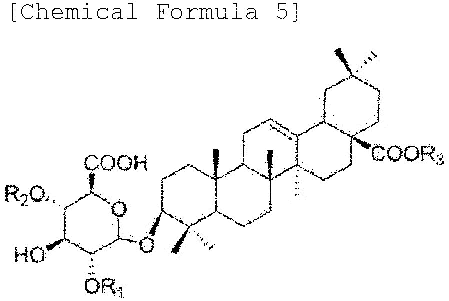

Another aspect of the present invention provides a pharmaceutical formulation comprising: (a) curcumin having a structure of Chemical Formula 1 or Chemical Formula 2 below or a pharmaceutically acceptable salt thereof; (b) any one ginsenoside selected from the group consisting of Chemical Formula 3, Chemical Formula 4 and Chemical Formula 5 below or a pharmaceutically acceptable salt thereof; and (c) a gelling polymer:

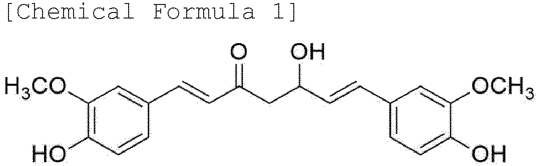

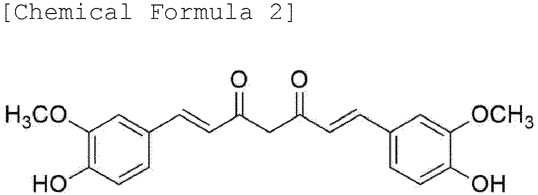

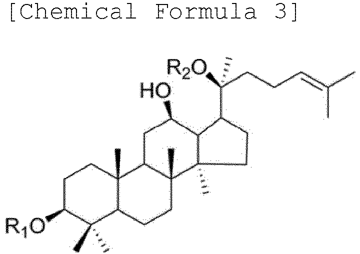

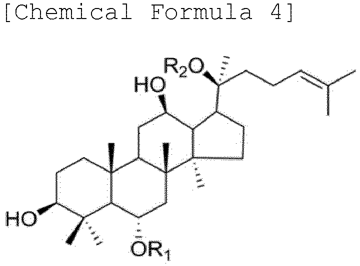

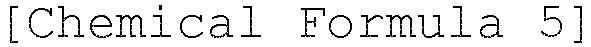

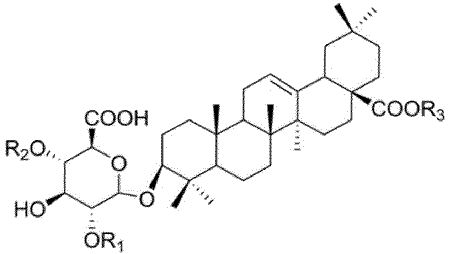

Another aspect of the present invention provides a health functional food composition comprising as active ingredients: curcumin having a structure of Chemical Formula 1 or Chemical Formula 2 below; and any one ginsenoside selected from the group consisting of Chemical Formula 3, Chemical Formula 4 and Chemical Formula 5 below:

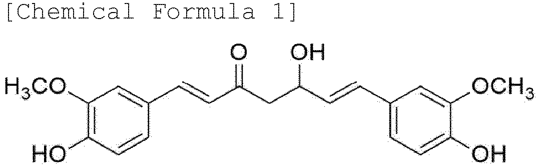

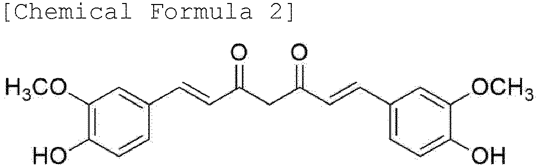

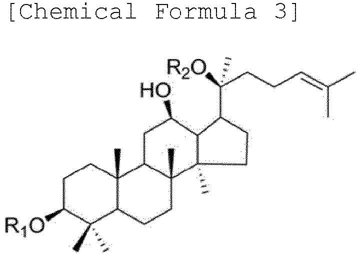

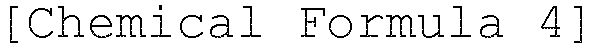

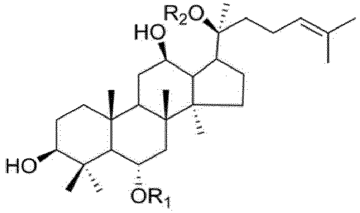

[Chemical Formula 5]

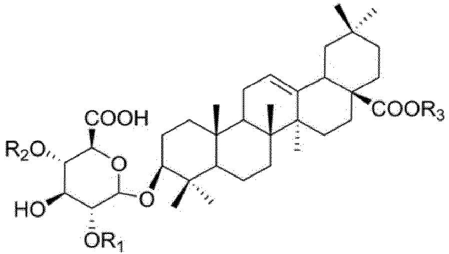

Another aspect of the present invention provides a pharmaceutical composition comprising as active ingredients: a curcuma extract or a turmeric extract; and a ginseng extract or a red ginseng extract.

Another aspect of the present invention provides a health functional food composition comprising as active ingredients: a curcuma extract or a turmeric extract; and a ginseng extract or a red ginseng extract.

Another aspect of the present invention provides a method for protecting against or alleviating radiation-induced damage, the method including administering to a subject the pharmaceutical composition above.

Another aspect of the present invention provides a method for preventing or treating an inflammatory bowel disease, the method including administering to a subject the pharmaceutical composition above.

Another aspect of the present invention provides a use of the pharmaceutical formulation above for the production of a medicament for alleviating or ameliorating radiation-induced damage or for preventing or treating an inflammatory bowel disease.

### ADVANTAGEOUS EFFECTS

The combined use of curcumin and ginsenoside according to the present invention restored cell growth, mobility, and endothelial cell tube-forming ability, reduced by irradiation, and inhibited an inflammatory response induced in macrophages by LPS treatment. In addition, damaged intestinal epithelial tissue was restored and the expression of inflammatory cytokines were suppressed in an irradiated mouse. Furthermore, a mixture of a ginseng extract and a turmeric extract restored damaged intestinal epithelial tissue in an irradiated mouse. Therefore, the combined use of curcumin and ginsenoside, or the combined use of a ginseng extract and a turmeric extract, can be utilized as a therapeutic agent for radiation proctitis.

### BRIEF DESCRIPTION OF THE DRAWINGS

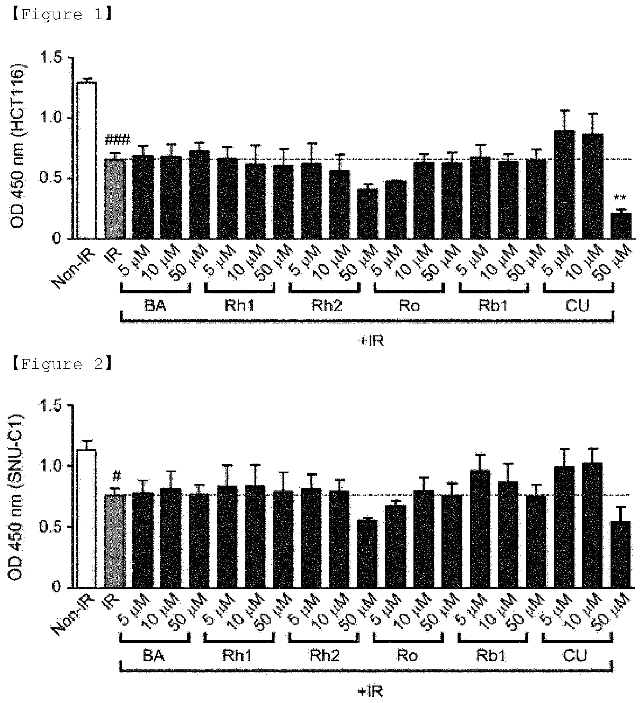

FIG. 1 is a graph showing the results of examining cell viability after treating an irradiated human colon cancer cell line (HCT116) with 5, 10, or 50 µM of butyric acid (BA), ginsenoside Rh1, Rh2, Rb1, Ro, or curcumin (CU). In this case, ###*p*<0.001 indicates significance relative to the non-irradiated group, and ***p*<0.01 indicates significance relative to the irradiated group. Non-IR: Non-irradiated, IR or +IR: Irradiated
FIG. 2 is a view showing the results of examining cell viability after treating an irradiated human colon cancer cell line (SNU-C1) with 5, 10 or 50 µM of butyric acid (BA), ginsenoside Rh1, Rh2, Rb1, Ro or curcumin (CU). In this case, #*p*<0.05 indicates significance relative to the non-irradiated group. Non-IR: Non-irradiated, IR or +IR: Irradiated

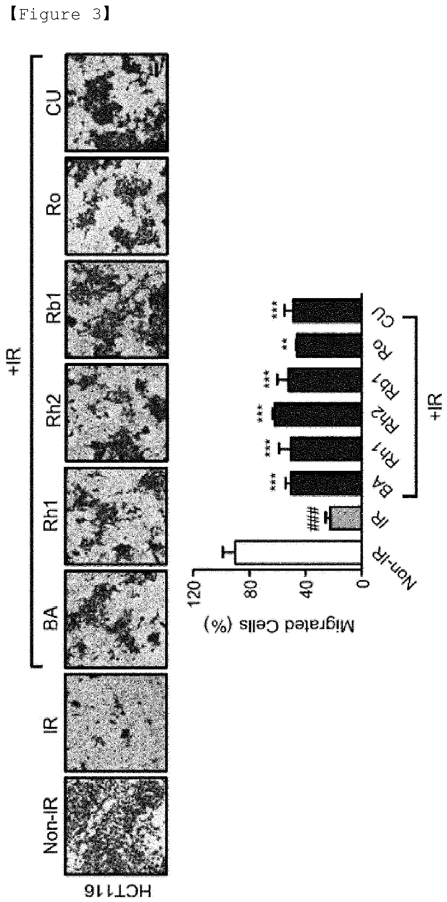

FIG. 3 is a view (top) and graph (bottom) showing the results of examining cell mobility after treating an irradiated human colon cancer cell line (HCT116) with 10 µM of butyric acid (BA), ginsenoside Rh1, Rh2, Rb1, Ro or curcumin (CU). In this case, ###*p*<0.001 indicates significance relative to the non-irradiated group, and ***p*<0.01 and ****p*<0.001 indicate significance relative to the irradiated group. Non-IR: Non-irradiated, IR or +IR: Irradiated, Scale bar=100 um

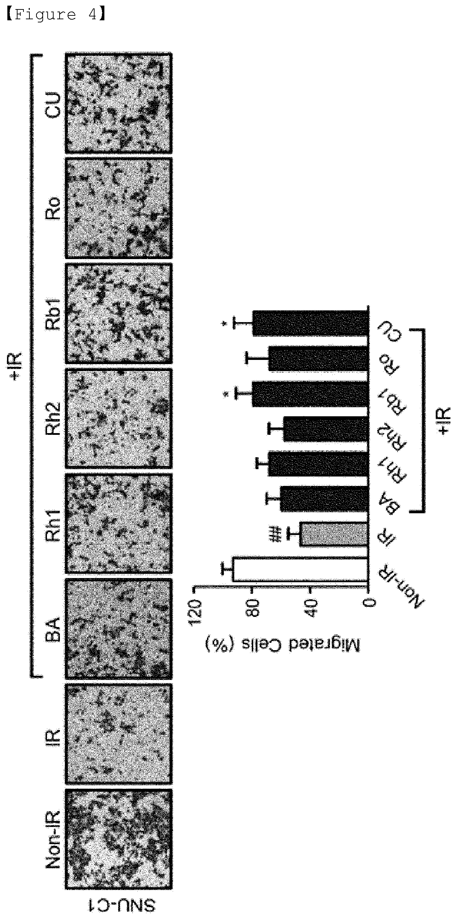

FIG. 4 is a view (top) and graph (bottom) showing the results of examining cell mobility after treating an irradiated human colon cancer cell line (SNU-C1) with 10 µM of butyric acid (BA), ginsenoside Rh1, Rh2, Rb1, Ro, or curcumin (CU) . In this case, ##*p*<0.01 indicates significance relative to the non-irradiated group, and **p*<0.05 indicates significance relative to the irradiated group. Non-IR: Non-irradiated, IR or +IR: Irradiated, Scale bar=100 µm

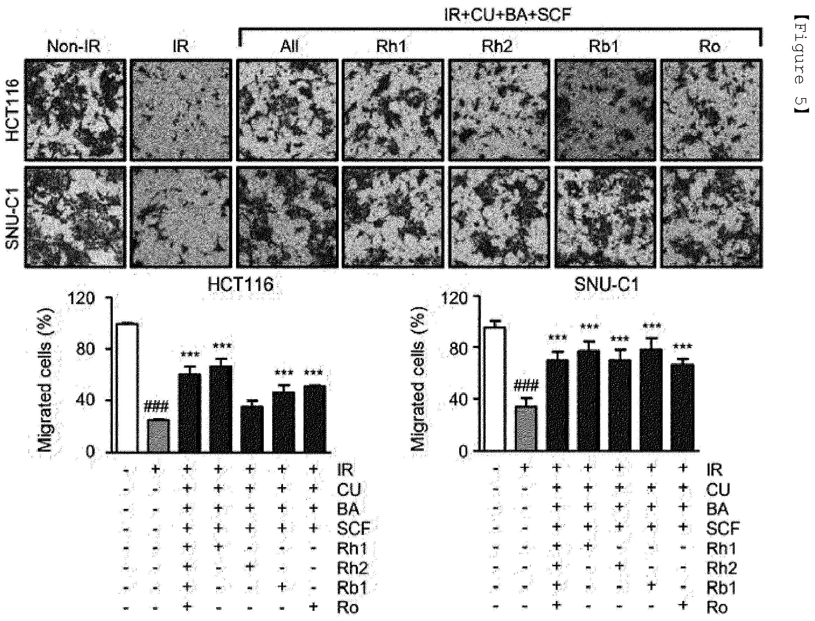

FIG. 5 is a view (top) and graph (bottom) showing the results of examining cell mobility after treating irradiated human colon cancer cell lines (HCT116 and SNU-C1) through combined use of curcumin (CU), butyric acid (BA), sucralfate (SCF), and ginsenoside. ###*p*<0.001 indicates significance relative to the non-irradiated group, and ****p*<0.001 indicates significance relative to the irradiated group. Non-IR: Non-irradiated, IR: Irradiated, Scale bar=100 um

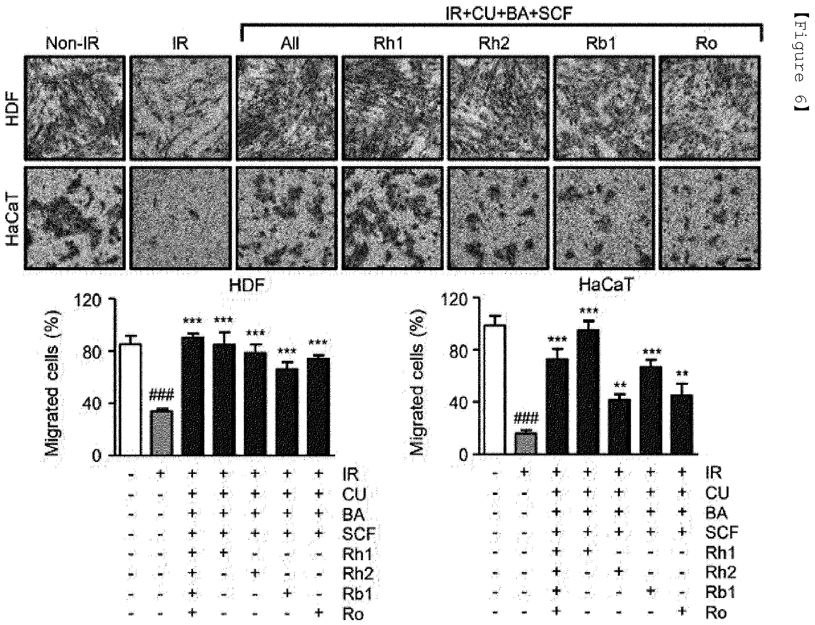

FIG. 6 is a view (top) and graph (bottom) showing the results of examining cell mobility after treating irradiated human dermal fibroblasts (HDF) and an irradiated human keratinocyte cell line (HaCaT) through combined use of curcumin (CU), butyric acid (BA), sucralfate (SCF), and ginsenoside. ###*p*<0.001 indicates significance relative to the non-irradiated group, and ***p*<0.01 and ****p*<0.001 indicate significance relative to the irradiated group. Non-IR: Non-irradiated, IR: Irradiated, Scale bar=100 µm

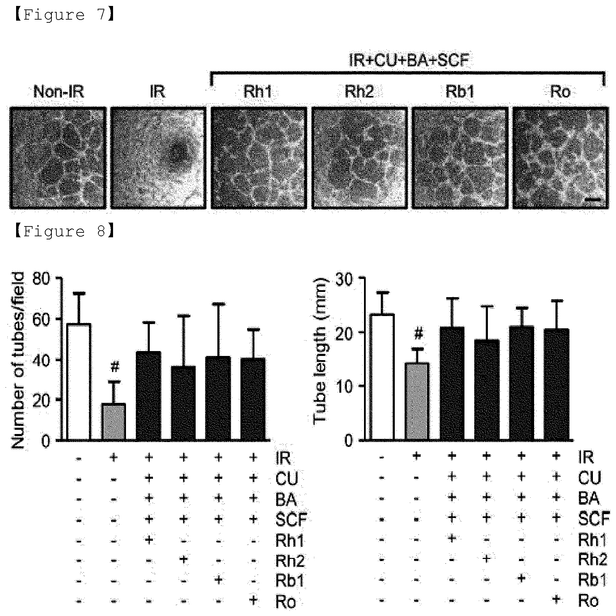

FIG. 7 is a view showing the results of observing tube formation after treating an irradiated human umbilical cord endothelial cell line (HUVEC) by using ginsenoside Rh1, Rh2, Rb1, or Ro in combination with curcumin (CU), butyric acid (BA), and sucralfate (SCF). In this case, the compounds were treated at a concentration of 10 µM. Non-IR: Non-irradiated, IR: Irradiated. Scale bar=300 µm.
FIG. 8 is a graph showing the results of measuring the number (left) and length (right) of tubes formed after treating an irradiated human umbilical vein endothelial cell line (HUVEC) by using ginsenoside Rh1, Rh2, Rb1, or Ro in combination with curcumin (CU), butyric acid (BA), and sucralfate (SCF). In this case, the compounds were treated at a concentration of 10 µM. #*p*<0.05 indicates significance relative to the non-irradiated group. Non-IR: Non-irradiated, IR: Irradiated

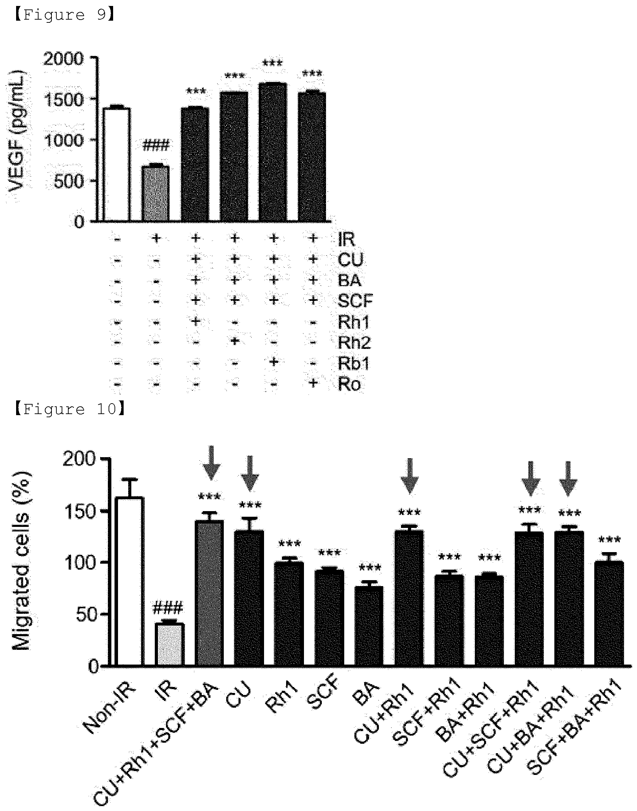

FIG. 9 is a graph showing the results of measuring the concentration of VEGF secreted into the medium after treating an irradiated human umbilical cord vascular endothelial cell line (HUVEC) by using ginsenoside Rh1, Rh2, Rb1 or Ro in combination with curcumin (CU), butyric acid (BA) and sucralfate (SCF). In this case, the compounds were treated at a concentration of 10 µM. ###*p*<0.001 indicates significance relative to the non-irradiated group, and ****p*<0.001 indicates significance relative to the irradiated group. Non-IR: Non-irradiated, IR: Irradiated
FIG. 10 is a graph showing the results of examining cell mobility after treating an irradiated human dermal fibroblast cell line (HDF) with ginsenoside Rh1, curcumin (CU), butyric acid (BA), and sucralfate (SCF) alone or in combination. In this case, the compounds were treated at a concentration of 10 µM. ###*p*<0.001 indicates significance relative to the non-irradiated group, and ****p*<0.001 indicates significance relative to the irradiated group. Non-IR: Non-irradiated, IR: Irradiated

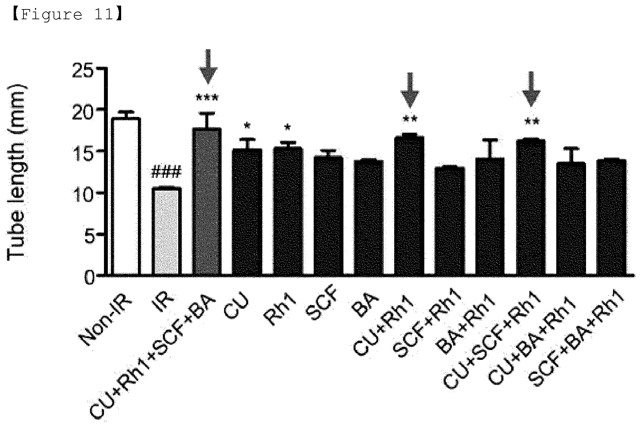

FIG. 11 is a graph showing the results of measuring the length of tubes formed after treating an irradiated human umbilical cord endothelial cell line (HUVEC) with ginsenoside Rh1, curcumin (CU), butyric acid (BA), and sucralfate (SCF) alone or in combination. In this case, the compounds were treated at a concentration of 10 µM. ###*p*<0.001 indicates significance relative to the non-irradiated group, and **p*<0.05 and ***p*<0.01 indicate significance relative to the irradiated group. Non-IR: Non-irradiated, IR: Irradiated

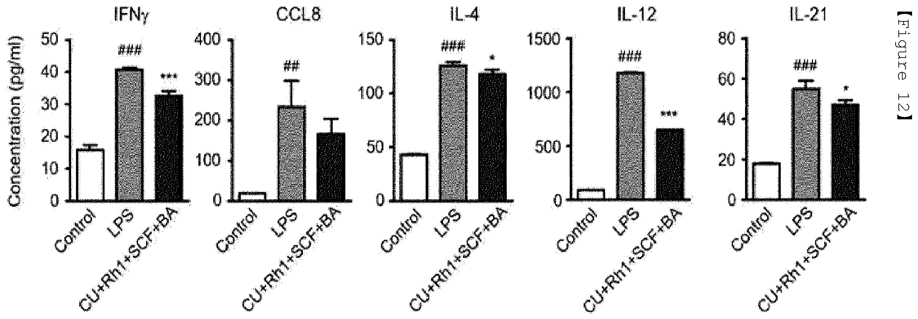

FIG. 12 is a view showing the results of measuring the amount of cytokines secreted into the culture medium after treating a human monocyte cell line (THP-1) treated with LPS, through combined use of ginsenoside Rh1, curcumin (CU), butyric acid (BA), and sucralfate (SCF). In this case, the compounds were treated at a concentration of 10 µM. ###*p*<0.001 indicates significance relative to the LPS untreatment group, and **p*<0.05 and ****p*<0.001 indicate significance relative to the LPS treatment group. LPS: lipopolysaccharide

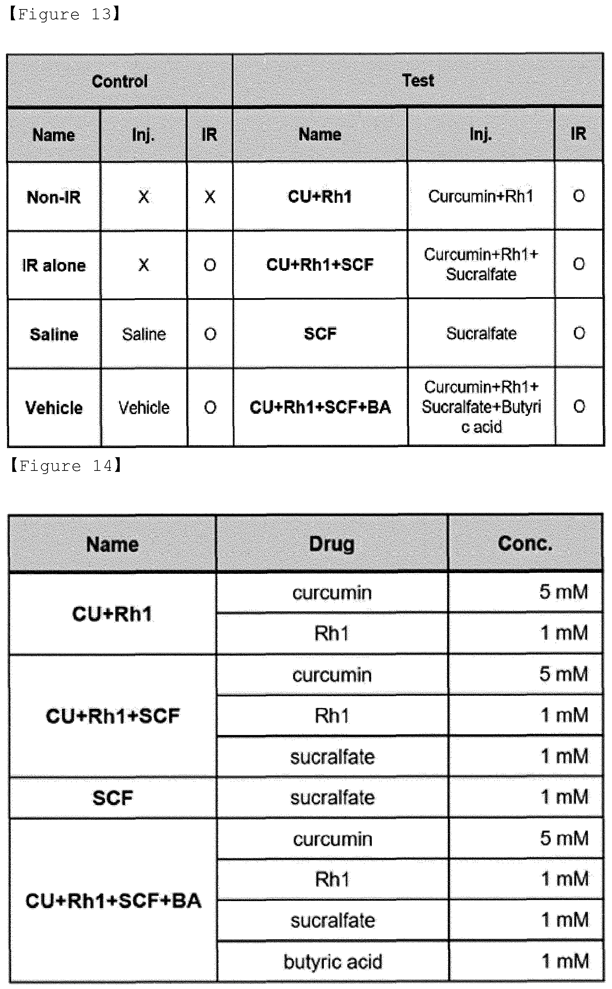

FIG. 13 is a table showing a test treatment group for treating a radiation proctitis model mouse through combined use of ginsenoside Rh1, curcumin (CU), butyric acid (BA), and sucralfate (SCF).
FIG. 14 is a table showing an effective drug composition and concentration of a test treatment group for treating a radiation proctitis model mouse through combined use of ginsenoside Rh1, curcumin (CU), butyric acid (BA), and sucralfate (SCF).

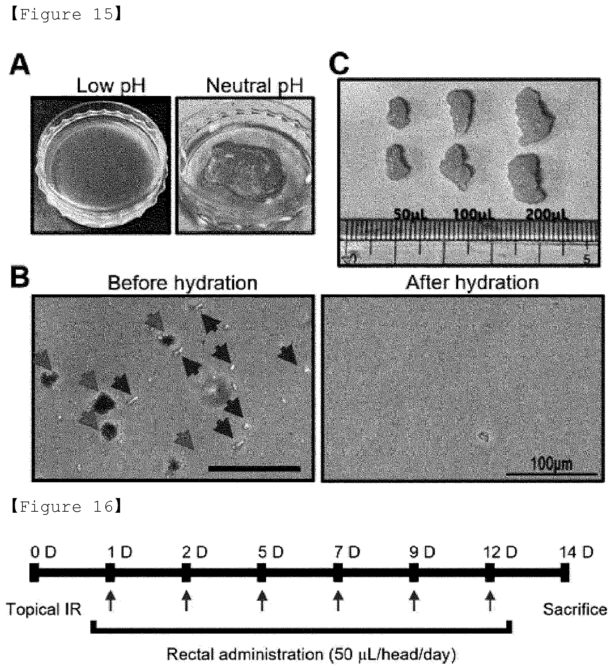

FIG. 15 is a view showing a formulation of a sample administered to a radiation proctitis model mouse.
FIG. 16 is a view showing an experimental schedule for examining the anti-inflammatory activity by co-administration of ginsenoside Rh1, curcumin (CU), butyric acid (BA), and sucralfate (SCF) in a radiation proctitis model mouse.

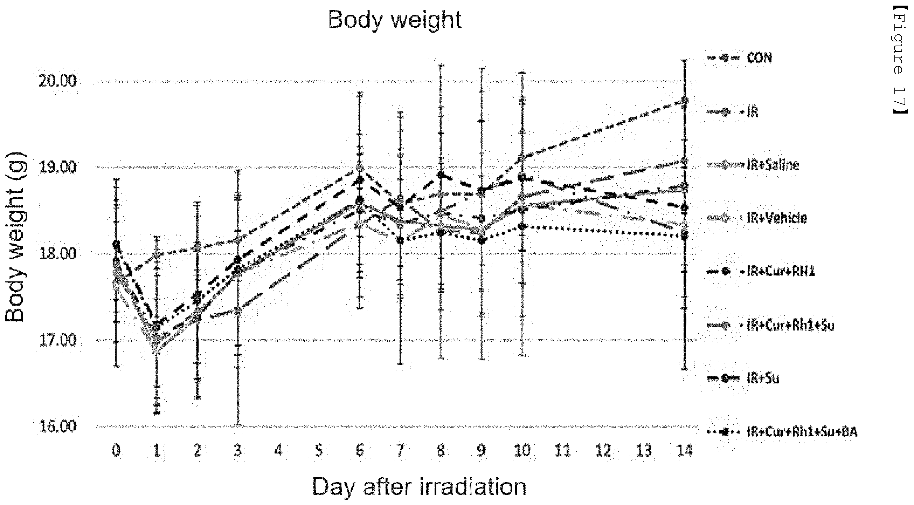

FIG. 17 is a graph showing the results of measuring the body weight during 14-day period of administration of sucralfate (SCF) alone, co-administration of curcumin (CU) and ginsenoside Rh1 (Rh1), co-administration of CU, Rh1, and SCF, and co-administration of Rh1, CU, butyric acid (BA), and SCF in a radiation proctitis model mouse. Non-IR: Non-irradiated, IR: Irradiated

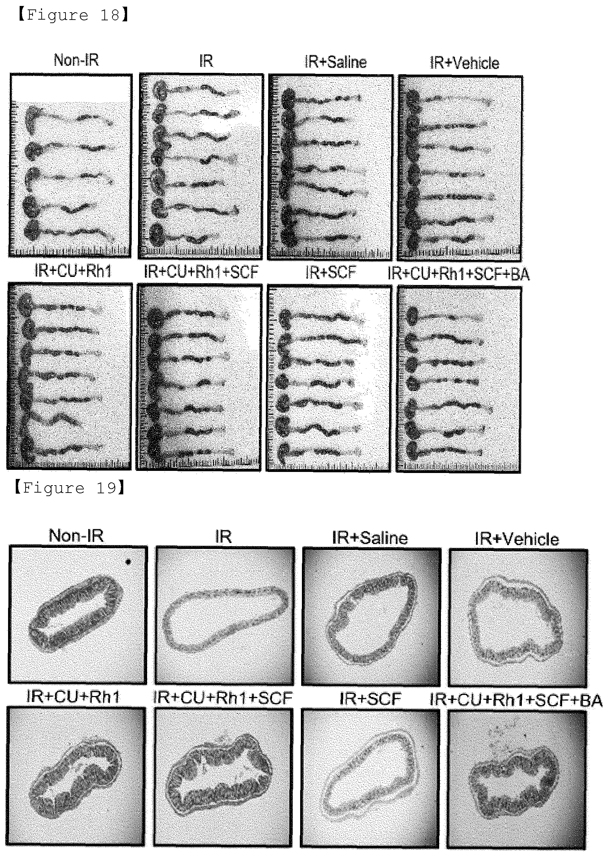

FIG. 18 is a view showing the results of observing the intestinal length after 14 days of administration of sucralfate (SCF) alone, co-administration of curcumin (CU) and ginsenoside Rh1 (Rh1), co-administration of CU, Rh1, and SCF, and co-administration of Rh1, CU, butyric acid (BA), and SCF in a radiation proctitis model mouse. Non-IR: Non-irradiated, IR: Irradiated.
FIG. 19 is a view showing the results of observing colon tissue at low magnification (X40) through H&E staining after 14 days of administration of sucralfate (SCF) alone, co-administration of curcumin (CU) and ginsenoside Rh1 (Rh1), co-administration of CU, Rh1 and SCF, and co-administration of Rh1, CU, butyric acid (BA), and SCF in a radiation proctitis model mouse.

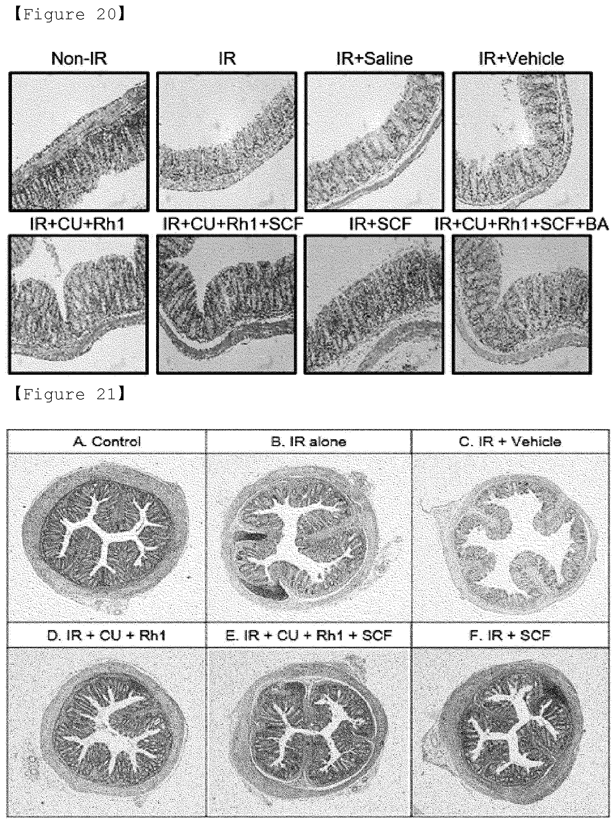

FIG. 20 is a view showing the results of observing colon tissue at high magnification (X200) through H&E staining after 14 days of administration of sucralfate (SCF) alone, co-administration of curcumin (CU) and ginsenoside Rh1 (Rh1), co-administration of CU, Rh1 and SCF, and co-administration of Rh1, CU, butyric acid (BA), and SCF in a radiation proctitis model mouse.
FIG. 21 is a view showing the results of observing rectal tissue at low magnification (X40) through H&E staining after 14 days of administration of sucralfate (SCF) alone, co-administration of curcumin (CU) and ginsenoside Rh1 (Rh1), and co-administration of CU, Rh1, and SCF in a radiation proctitis model mouse.

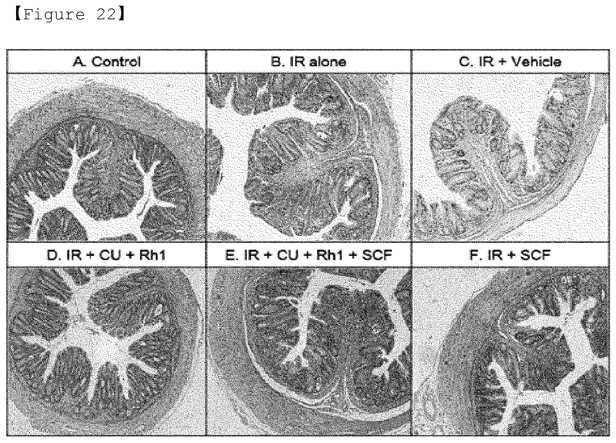

FIG. 22 is a view showing the results of observing rectal tissue at high magnification (X200) through H&E staining after 14 days of administration of sucralfate (SCF) alone, co-administration of curcumin (CU) and ginsenoside Rh1 (Rh1), and co-administration of CU, Rh1, and SCF in a radiation proctitis model mouse.

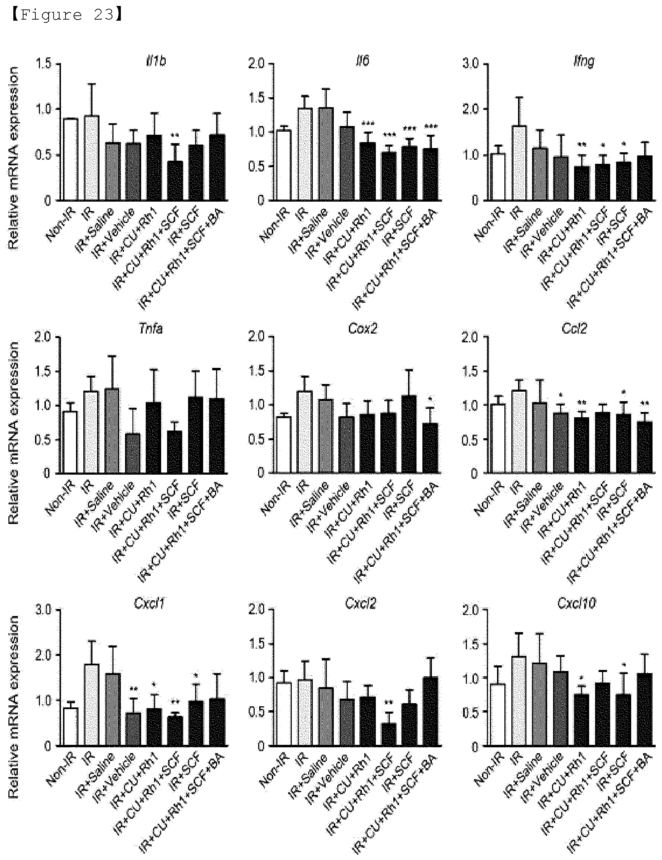

FIG. 23 is a graph showing the results of analyzing the expression of inflammatory cytokines in colon tissue through qPCR after 14 days of administration of sucralfate (SCF) alone, co-administration of curcumin (CU) and ginsenoside Rh1 (Rh1), co-administration of CU, Rh1, and SCF, and co-administration of Rh1, CU, butyric acid (BA), and SCF in a radiation proctitis model mouse. **p*<0.05, ***p*<0.01, and ****p*<0.001 indicate significance relative to the irradiated group. Non-IR: Non-irradiated, IR: Irradiated

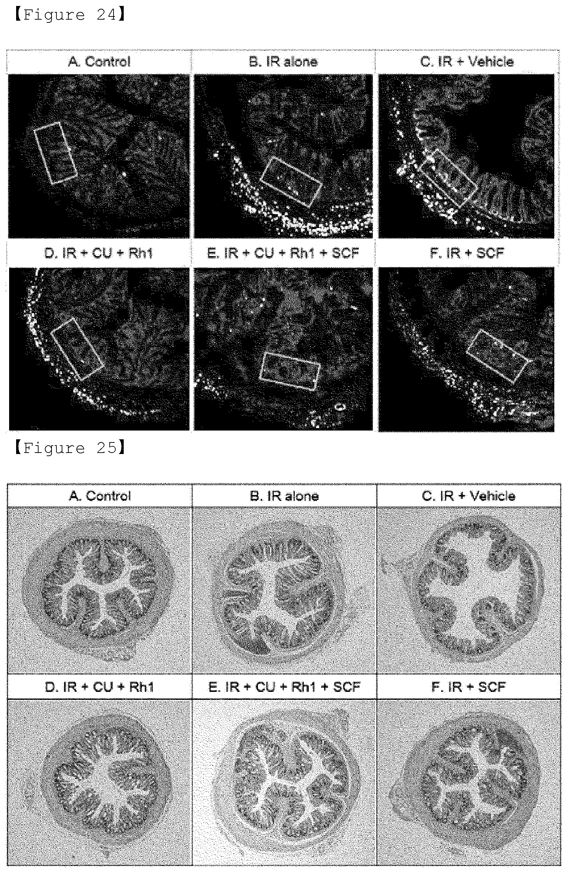

FIG. 24 is a view showing the results of observing apoptosis in rectal tissue through TUNEL staining at low magnification (X40) after 14 days of administration of sucralfate (SCF) alone, co-administration of curcumin (CU) and ginsenoside Rh1 (Rh1), and co-administration of CU, Rh1, and SCF in a radiation proctitis model mouse.
FIG. 25 is a view showing the results of observing the expression level of PCNA in rectal tissue through immunohistochemistry at low magnification (X40) after 14 days of administration of sucralfate (SCF) alone, co-administration of curcumin (CU) and ginsenoside Rh1 (Rh1), and co-administration of CU, Rh1, and SCF in a radiation proctitis model mouse.

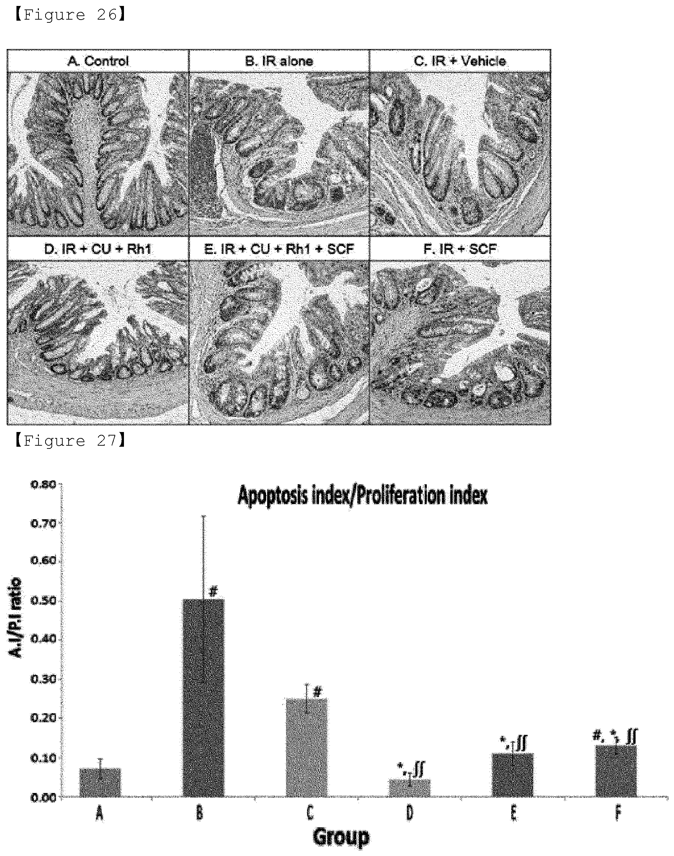

FIG. 26 is a view showing the results of observing the expression level of PCNA in rectal tissue through immunohistochemistry at high magnification (X200) after 14 days of administration of sucralfate (SCF) alone, co-administration of curcumin (CU) and ginsenoside Rh1 (Rh1), and co-administration of CU, Rh1, and SCF in a radiation proctitis model mouse.
FIG. 27 is a graph showing the results of analyzing the ratio of PCNA (proliferating cell nuclear antigen) expression (cell proliferation) and apoptosis in rectal tissue through TUNEL staining and immunohistochemistry after 14 days of administration of sucralfate (SCF) alone, co-administration of curcumin (CU) and ginsenoside Rh1 (Rh1), and co-administration of CU, Rh1, and SCF in a radiation proctitis model mouse. In this case, #*p*<0.05 indicates significance relative to the non-irradiated group, **p*<0.05 indicates significance relative to the irradiated group, and ^{∫∫}p<0.01 indicates significance relative to the vehicle-administered group. A: control, B: IR administration alone, C: IR+vehicle, D: IR+CD+Rh1, E: IR+CD+Rh1+SCF, F: IR+SCF

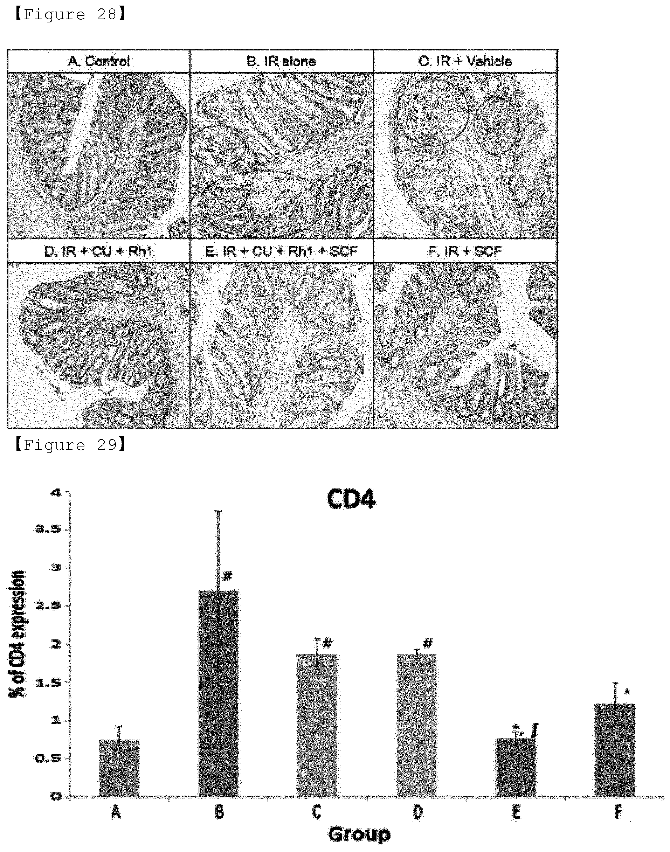

FIG. 28 is a view showing the results of examining the infiltration of T cells in tissue by observing the expression level of CD4 in rectal tissue through immunohistochemistry at high magnification (X200) after 14 days of administration of sucralfate (SCF) alone, co-administration of curcumin (CU) and ginsenoside Rh1 (Rh1), and co-administration of CU, Rh1, and SCF in a radiation proctitis model mouse.
FIG. 29 is a graph showing the quantification of the expression of CD4 (used as an indicator of T cell infiltration) in rectal tissue observed through immunohistochemistry after 14 days of administration of sucralfate (SCF) alone, co-administration of curcumin (CU) and ginsenoside Rh1 (Rh1), and co-administration of CU, Rh1, and SCF in a radiation proctitis model mouse. In this case, #*p*<0.05 indicates significance relative to the non-irradiated group, **p*<0.05 indicates significance relative to the irradiated group, and ^{∫}p<0.05 indicates significance relative to the vehicle-administered group. A: control, B: IR administration alone, C: IR+vehicle, D: IR+CD+Rh1, E: IR+CD+Rh1+SCF, F: IR+SCF

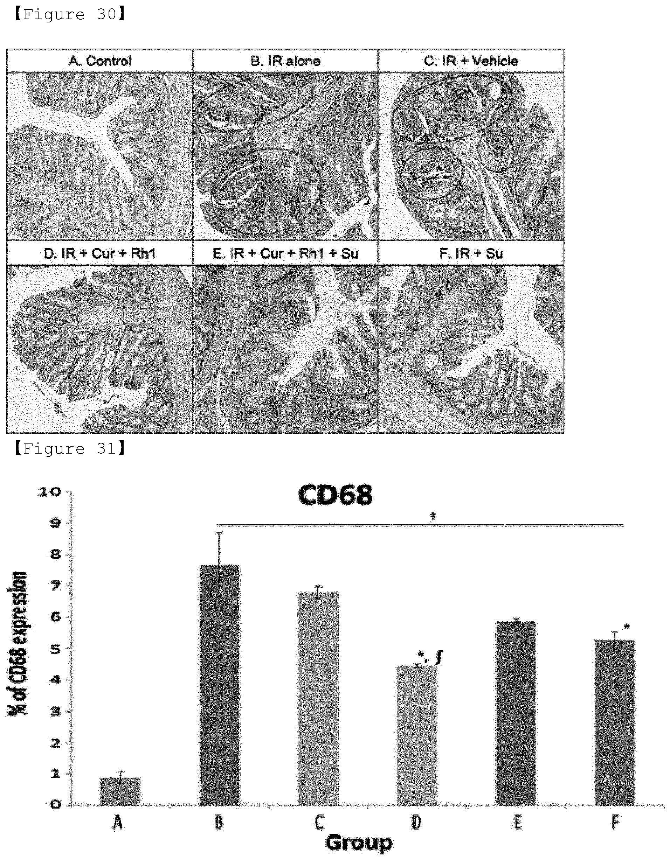

FIG. 30 is a view showing the results of examining the infiltration of macrophages in tissue by observing the expression level of CD68 in rectal tissue through immunohistochemistry at high magnification (X200) after 14 days of administration of sucralfate (SCF) alone, co-administration of curcumin (CU) and ginsenoside Rh1 (Rh1), and co-administration of CU, Rh1, and SCF in a radiation proctitis model mouse.
FIG. 31 is a graph showing the quantification of the expression of CD68 (used as an indicator of macrophage infiltration) in rectal tissue observed through immunohistochemistry after 14 days of administration of sucralfate (SCF) alone, co-administration of curcumin (CU) and ginsenoside Rh1 (Rh1), and co-administration of CU, Rh1, and SCF in a radiation proctitis model mouse. In this case, #*p*<0.05 indicates significance relative to the non-irradiated group, **p*<0.05 indicates significance relative to the irradiated group, and ^{∫}p<0.05 indicates significance relative to the vehicle-administered group. A: control, B: IR administration alone, C: IR+vehicle, D: IR+CD+Rh1, E: IR+CD+Rh1+SCF, F: IR+SCF

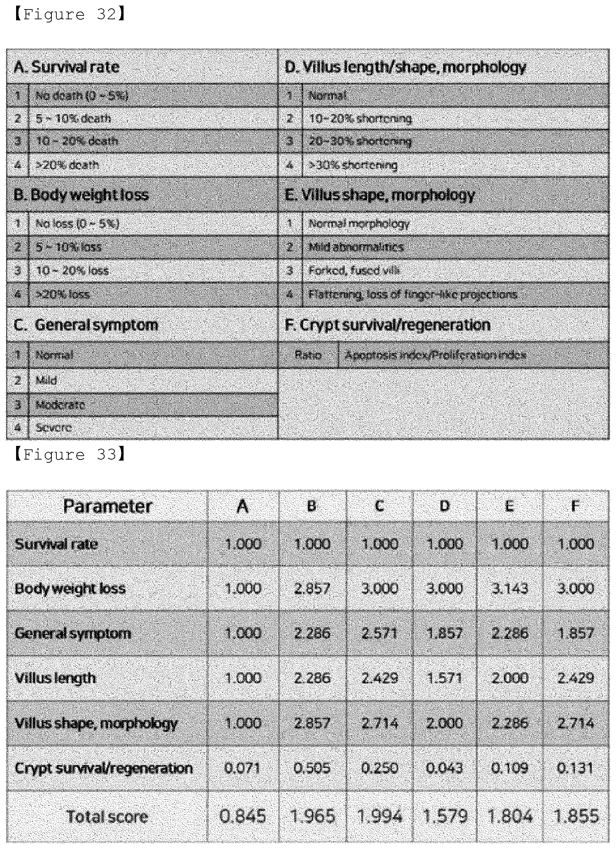

FIG. 32 is a view showing the scoring criteria for survival rate, body weight change, general symptom observation, intestinal villus length, intestinal villus shape, and intestinal secretory gland regeneration in radiation injury score (RIS) evaluation.
FIG. 33 is a view showing the results of RIS evaluation for each experimental group after 14 days of administration of sucralfate (SCF) alone, co-administration of curcumin (CU) and ginsenoside Rh1 (Rh1), and co-administration of CU, Rh1, and SCF in a radiation proctitis model mouse. A: control, B: IR administration alone, C: IR+vehicle, D: IR+CD+Rh1, E: IR+CD+Rh1+SCF, F: IR+SCF

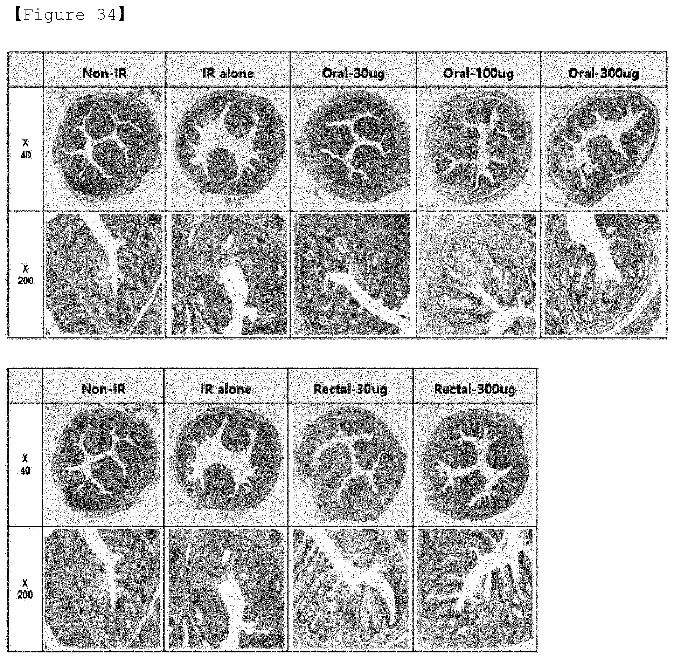

FIG. 34 is a view showing the results of observing rectal tissue through H&E staining after 14 days of oral or rectal administration of a mixture of a ginseng extract and a turmeric extract in a radiation proctitis model mouse.

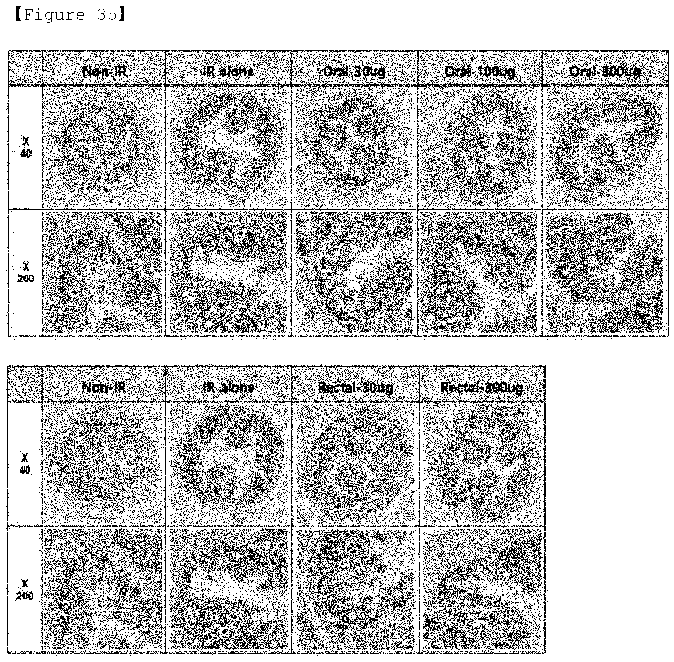

FIG. 35 is a view showing the results of examining PCNA expression in rectal tissue through immunohistochemistry after 14 days of oral or rectal administration of a mixture of a ginseng extract and a turmeric extract in a radiation proctitis model mouse.

### BEST MODE FOR CARRYING OUT THE INVENTION

### Pharmaceutical composition

### Pharmaceutical composition including curcumin and ginsenoside

An aspect of the present invention provides a pharmaceutical composition for protecting against or alleviating radiation-induced damage, comprising curcumin and ginsenoside as active ingredients.

Another aspect of the present invention provides a pharmaceutical composition for protecting against or alleviating radiation-induced damage, comprising as active ingredients: curcumin having a structure of Chemical Formula 1 or Chemical Formula 2 below or a pharmaceutically acceptable salt thereof; and any one ginsenoside selected from the group consisting of Chemical Formula 3, Chemical Formula 4 and Chemical Formula 5 below or a pharmaceutically acceptable salt thereof:

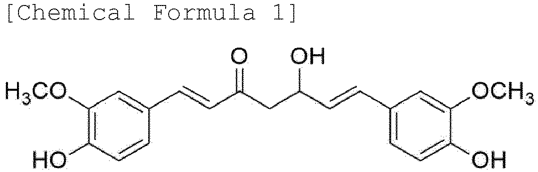

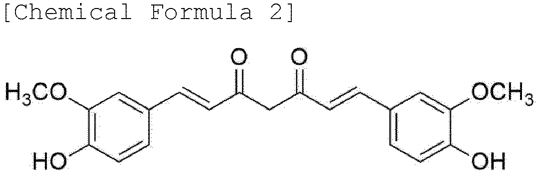

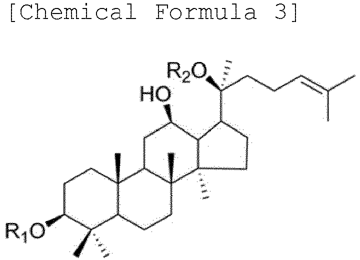

In the formula above, R₁ may be any one selected from the group consisting of H-, Glu- and Glu-Glu-, and R₂ may be any one selected from the group consisting of H-, Glu-, Glu-Glu-, Arap-Glu- and Araf-Glu-.

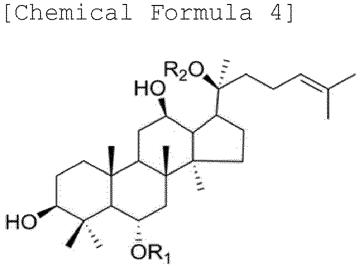

In the formula above, R₁ may be any one selected from the group consisting of H-, Glu-, Rha-Glu- and Xyl-Glu, and R₂ may be H- or Glu-,

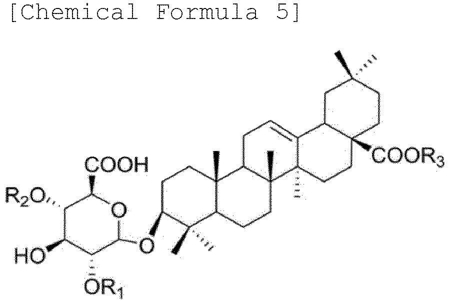

In the formula above, R₁ and R₃ are Glu-, and R₂ is H-. In Chemical Formulas 3 to 5, Glu is β-D-glucopyranosyl, Arap is α-L-arabinopyranosyl, Araf is α-L-arabinofuranosyl, Xyl is β-D-xylopyranosyl, and Rha is α-L-rhamnopyranosyl.

The term "radiation" as used herein refers to a stream of particles moving at high speed and electromagnetic waves with a short wavelength. The radiation may include, but is not limited to, α-rays, β-rays, γ-rays, and a flux of protons, neutrons, and heavy atomic nuclei. In a specific example of the present invention, radiation causing damage due to irradiation may be, for example, ionizing radiation (IR) .

The term "radiation-induced damage" as used herein refers to a phenomenon in which damage to blood vessels or tissue, tissue inflammation, tissue fibrosis, or the like is induced by radiation exposure. The radiation-induced damage may be a radiation treatment side effect that occurs when even normal tissue is exposed to radiation during radiation treatment for cancer, but is not limited thereto.

The term "protecting against radiation-induced damage" as used herein refers to suppression or reduction of any radiation-induced damage caused by radiation exposure which is applied to a living body before or after radiation exposure. In addition, "alleviating radiation-induced damage" means suppression or reduction of any radiation-induced damage caused by radiation exposure which is applied to a living body within a short period of time after radiation exposure before the palpable signs of radiation exposure appear.

The term "curcumin" as used herein is curcuminoid of turmeric, a polyphenol component-containing yellow spice which is extracted from the root of *Curcuma longa Linn* (Zingiberaceae), a plant belonging to the ginger family in East India, and which is widely used in Indian food. Curcumin is used as a yellow pigment in food additives and is used as a spice. Curcumin has various functions in cell physiology and is known to be related to many cellular components including inflammation-related proteins, cell signaling substances, transcription factors, and the like. In particular, the anti-inflammatory and anticancer effects of curcumin have been studied extensively. Curcumin has a molecular formula of C₂₁H₂₀O₆ and exists in a Diketo form of Chemical Formula 1 and a Keto-enol form of Chemical Formula 2.

The term "ginsenoside" used herein is a type of saponin, a type of glycoside, derived from the roots, stems, leaves, barks, seeds, etc., of plants and is also contained in the bodies of some echinoderms (starfish, sea cucumbers). Saponin is known as a component that enhances the immunity of the human body, and ginsenoside, which is a type of saponin and contained in ginseng, red ginseng, mountain ginseng, wild ginseng, and black ginseng, etc. is particularly famous. There are various types of ginsenosides, including ginsenoside Rg1, Rg2, Rg3, Rg4. Rg6, Rb1, Rb2, Rh1, Rh2, Rc, Rd, Re, and Ro, and the types and contents of ginsenosides may vary depending on plant types, cultivation conditions, extraction methods, and parts of the ginseng, red ginseng, or mountain ginseng.

In one embodiment of the present invention, the pharmaceutical composition may comprise ginsenosides Rh1, Rh2, Rb, Ro, or a combination thereof. In this case, the ginsenoside Rh1 has a structure of Chemical Formula 6, Rh2 has a structure of Chemical Formula 7, Rb1 has a structure of Chemical Formula 8, and Ro has a structure Chemical Formula 9:

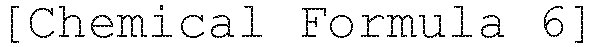

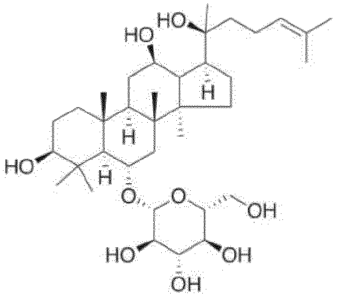

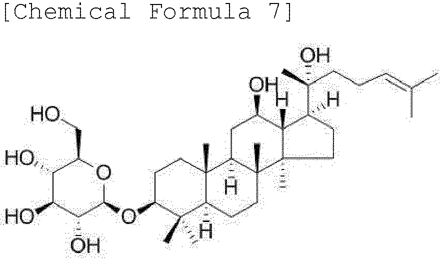

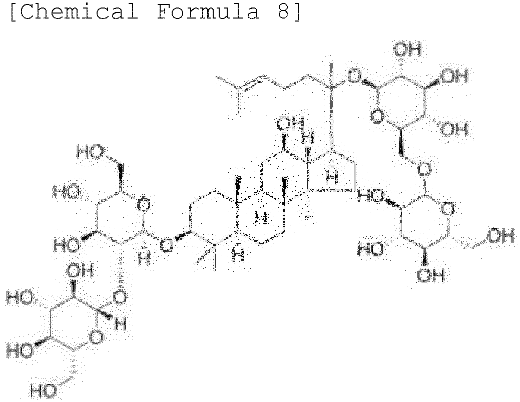

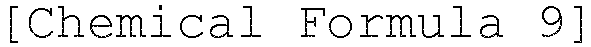

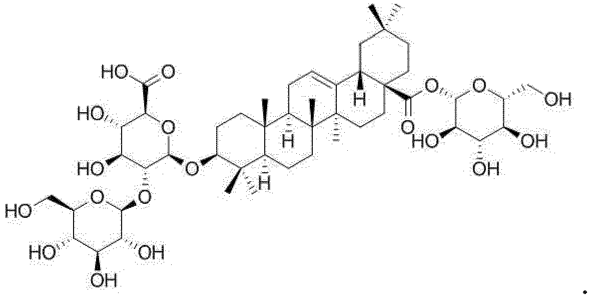

The pharmaceutical composition may contain, as an active ingredient, the curcumin or a pharmaceutically acceptable salt thereof in an amount of 0.001 wt% to 20 wt% with respect to the total weight of the composition. Specifically, the pharmaceutical composition may contain the curcumin or a pharmaceutically acceptable salt thereof in an amount of about 0.03% (w/v) to about 0.4% (w/v) with respect to the total weight of the composition. More specifically, the curcumin or a pharmaceutically acceptable salt thereof may be included in an amount of about 0.03% (w/v) to about 0.4% (w/v), about 0.04% (w/v) to about 0.3% (w/v), about 0.05% (w/v) to about 0.2% (w/v), or about 0.06% (w/v) to about 0.1% (w/v) with respect to the total weight of the composition.

In addition, the pharmaceutical composition may contain, as an active ingredient, the ginsenoside or a pharmaceutically acceptable salt thereof in an amount of 0.001 wt% to 20 wt% with respect to the total weight of the composition. Specifically, the pharmaceutical composition may contain the ginsenoside or a pharmaceutically acceptable salt thereof in an amount of about 0.01% (w/v) to about 0.2% (w/v) with respect to the total weight of the composition. More specifically, the ginsenoside or a pharmaceutically acceptable salt thereof may be included in an amount of about 0.01% (w/v) to about 0.2% (w/v), about 0.02% (w/v) to about 0.1% (w/v), about 0.03% (w/v) to about 0.08% (w/v), or about 0.04% (w/v) to about 0.06% (w/v) with respect to the total weight of the composition.

The curcumin or a pharmaceutically acceptable salt thereof and the ginsenoside or a pharmaceutically acceptable salt thereof may be in the form of a mixture, and the mixing ratio of the curcumin or a pharmaceutically acceptable salt thereof and the ginsenoside or a pharmaceutically acceptable salt thereof may be 10:1 to 1:10.

In this case, the ginsenoside may include Rh1, Rh2, Rb, Ro, or a mixture of all thereof, but is not limited thereto. Ginsenoside Rh1, Rh2, Rb, and Ro are the same as described above.

The term "pharmaceutically acceptable salt" used herein means a salt having a pharmaceutically usable form among salts which are materials in which cations and anions are combined by electrostatic attraction, and means any organic or inorganic addition salt of the compound having a concentration that is relatively non-toxic and has harmless effective actions on a patient while not reducing the beneficial efficacy of the compound. Such a salt includes acid addition salts formed by pharmaceutically acceptable free acids or metal salts formed by bases.

Organic acids and inorganic acids may be used as free acids, hydrochloric acid, phosphoric acid, sulfuric acid, nitric acid, tartaric acid and the like may be used as inorganic acids, and methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid and the like may be used as organic acids, but the free acids are not limited thereto.

In addition, a base may be used to prepare a pharmaceutically or food-wise acceptable metal salt. An alkali metal salt or an alkaline earth metal salt is obtained, for example, by dissolving a compound in an excessive amount of an alkali metal hydroxide or an alkaline earth metal hydroxide solution, filtering out the undissolved compound salt, and then evaporating and drying the filtrate. In this case, sodium, potassium, or calcium salts are particularly suitable for pharmaceutical or food purposes, but are not limited thereto. In addition, a silver salt corresponding thereto may be obtained by reacting an alkali metal or alkaline earth metal salt with a suitable silver salt (e.g., silver nitrate).

The pharmaceutical composition of the present invention may further include at least one compound selected from the group consisting of sulfasalazine, mesalazine (5-aminosalicytic acid, 5-ASA), sucralfate, pentosan polysulfate (PPS), butyric acid, prednisolone, azathioprine, tofacitinib, ruscogenin, and pentoxifylline.

The sulfasalazine is used as a therapeutic agent for inflammatory bowel disease and is accompanied by side effects such as nausea, vomiting, loss of appetite, headache, hair loss, hepatitis, pneumonia, enteritis, and sperm reduction. The sulfasalazine has a structure in which an aminosalicylate known as 5-aminosalicytic acid (5-ASA) is attached to a sulfa-group molecule, wherein the part that shows the therapeutic effect is 5-ASA, and the sulfa-group is known to be involved in the side effects.

Mesalazine (5-aminosalicytic acid, 5-ASA) is a drug developed to reduce the side effects of sulfasalazine, and has a form in which a sulfapyridine molecule is removed from sulfasalazine. Mesalazine is used as an inflammatory bowel disease treatment in the form of oral medication, suppositories, and enema solutions.

Sucralfate is a drug used to treat gastric ulcers, gastroesophageal reflux disease, radiation proctitis, gastritis, etc., and prevent stress ulcers. Sucralfate is used in oral and suppository forms, and accompanied by side effects such as constipation, bezoar formation, headache, and dry mouth.

Pentosan polysulfate (PPS) is a drug used for interstitial cystitis/cystic pain syndrome (IC/PBS) therapeutic agent, and is known to have side effects such as diarrhea, heartburn, abdominal pain, and gastrointestinal disorders.

Butyric acid is a metabolite produced by intestinal bacteria and is a type of short-chain fatty acid. Butyric acid is known to have anti-inflammatory effects, intestinal bactericidal effects, and blood circulation-improving effects.

Prednisolone is a steroid hormone synthesized in the adrenal cortex, used as an immunosuppressant and anti-inflammatory agent, and is mainly used to treat lupus, psoriasis, and ulcerative colitis.

Azathioprine, which is an immunosuppressant, is used for rheumatoid arthritis, granulomatosis with polyangiitis, Crohn's disease, ulcerative colitis, and organ transplantation to prevent rejection. Common side effects include bone marrow suppression, vomiting, etc.

Tofacitinib is a JAK inhibitor used to treat rheumatoid arthritis, psoriatic arthritis, and ulcerative colitis. Common side effects include diarrhea, headache, and hypertension, and serious side effects include infection, cancer, and pulmonary embolism.

Ruscogenin is a steroid sapogenin found in *Ophiopogon japonicus,* a traditional Chinese herb. Physiological activities thereof have been reported, including anti-inflammation, prevention of hemorrhoids, inhibition of platelet aggregation, reduction of pulmonary hypertension, relief of acute lung injury, and attenuation of liver damage.

Pentoxifylline, also known as oxpentifyline, is a xanthine derivative used as a therapeutic agent for muscle pain in people with peripheral arterial disease. The effect thereof in treating alcoholic hepatitis has also been reported.

In one embodiment of the present invention, the pharmaceutical composition may further include sucralfate and/or butyric acid. In this case, the pharmaceutical composition may contain sucralfate or butyric acid in an amount of 0.001 wt% to 20 wt%, respectively, with respect to the total weight of the composition.

Specifically, the sucralfate may be contained in an amount of about 0.02% (w/v) to about 0.5% (w/v) with respect to the total weight of the composition. More specifically, the sucralfate may be contained in an amount of about 0.02% (w/v) to about 0.5% (w/v), about 0.03% (w/v) to about 0.4% (w/v), about 0.04% (w/v) to about 0.3% (w/v), about 0.05% (w/v) to about 0.2% (w/v), or about 0.06% (w/v) to about 0.1% (w/v).

Specifically, the butyric acid may be contained in an amount of about 0.0002% (w/v) to about 0.02% (w/v) with respect to the total weight of the composition. More specifically, the butyric acid may be contained in an amount of about 0.0002% (w/v) to about 0.02% (w/v), about 0.0004% (w/v) to about 0.01% (w/v), about 0.0008% (w/v) to about 0.008% (w/v), or about 0.001% (w/v) to about 0.004% (w/v).

The pharmaceutical composition may include a mixture of curcumin or a pharmaceutically acceptable salt thereof, ginsenoside or a pharmaceutically acceptable salt thereof, and sucralfate. The mixing ratio of curcumin or a pharmaceutically acceptable salt thereof, ginsenoside or a pharmaceutically acceptable salt thereof, and sucralfate may be 10:1:1 to 1:1:1.

The pharmaceutical composition may include a mixture of curcumin or a pharmaceutically acceptable salt thereof, ginsenoside or a pharmaceutically acceptable salt thereof, and butyric acid. The mixing ratio of curcumin or a pharmaceutically acceptable salt thereof, ginsenoside or a pharmaceutically acceptable salt thereof, and butyric acid may be 21:1:1 to 1:1:1.

The pharmaceutical composition may include a mixture of curcumin or a pharmaceutically acceptable salt thereof, ginsenoside or a pharmaceutically acceptable salt thereof, butyric acid, and sucralfate. The mixing ratio of curcumin or a pharmaceutically acceptable salt thereof, ginsenoside or a pharmaceutically acceptable salt thereof, butyric acid and sucralfate may be 21:1:1:1 to 1:1:1:1.

The pharmaceutical composition of the present invention is used to protect tissue exposed to radiation or promoting recovery of damaged areas by suppressing inflammatory reactions in tissue caused by radiation exposure.

Therefore, the pharmaceutical composition may be administered before or after radiation exposure to protect tissue from damage caused by radiation or to cure damaged tissue. Preferably, the pharmaceutical composition may be administered after radiation exposure in radiation therapy, but is not limited thereto.

Another aspect of the present invention provides a pharmaceutical composition for preventing or treating an inflammatory bowel disease, comprising as active ingredients: curcumin having a structure of Chemical Formula 1 or Chemical Formula 2 below or a pharmaceutically acceptable salt thereof; and any one ginsenoside selected from the group consisting of Chemical Formula 3, Chemical Formula 4 and Chemical Formula 5 below or a pharmaceutically acceptable salt thereof:

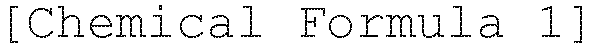

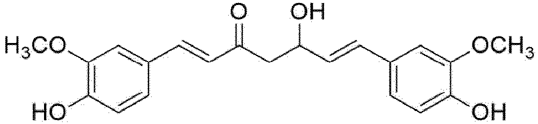

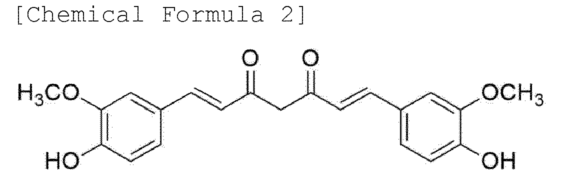

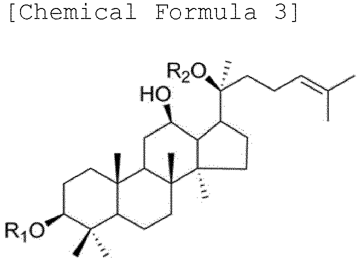

In the formula above, R₁ may be any one selected from the group consisting of H-, Glu- and Glu-Glu-, and R₂ may be any one selected from the group consisting of H-, Glu-, Glu-Glu-, Arap-Glu- and Araf-Glu-.

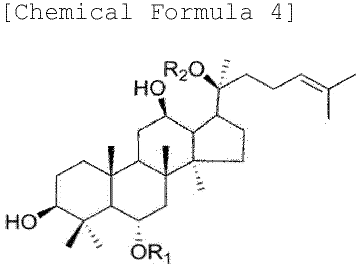

In the formula above, R₁ may be any one selected from the group consisting of H-, Glu-, Rha-Glu- and Xyl-Glu, and R₂ may be H- or Glu-.

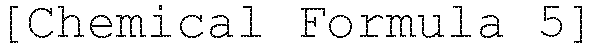

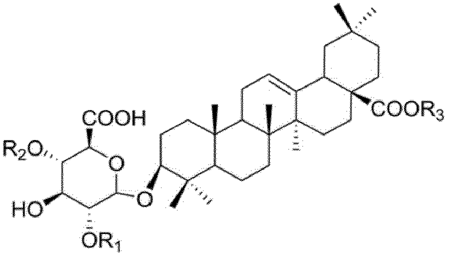

In the formula above, R₁ and R₃ is Glu-, and R₂ is H-.

In Chemical Formulas 3 to 5, Glu is β-D-glucopyranosyl, Arap is α-L-arabinopyranosyl, Araf is α-L-arabinofuranosyl, Xyl is β-D-xylopyranosyl, and Rha is α-L-rhamnopyranosyl.

Here, the curcumin, the ginsenoside and the pharmaceutically acceptable salt are the same as described above.

The pharmaceutical composition may contain, as an active ingredient, the curcumin or a pharmaceutically acceptable salt thereof in an amount of 0.001 wt% to 20 wt% with respect to the total weight of the composition. Specifically, the pharmaceutical composition may contain the curcumin or a pharmaceutically acceptable salt thereof in an amount of about 0.03% (w/v) to about 0.4% (w/v) with respect to the total weight of the composition. More specifically, the curcumin or a pharmaceutically acceptable salt thereof may be included in an amount of about 0.03% (w/v) to about 0.4% (w/v), about 0.04% (w/v) to about 0.3% (w/v), about 0.05% (w/v) to about 0.2% (w/v), or about 0.06% (w/v) to about 0.1% (w/v) with respect to the total weight of the composition. In addition, the pharmaceutical composition may contain, as an active ingredient, the ginsenoside or a pharmaceutically acceptable salt thereof in an amount of 0.001 wt% to 20 wt% with respect to the total weight of the composition. Specifically, the pharmaceutical composition may contain the ginsenoside or a pharmaceutically acceptable salt thereof in an amount of about 0.01% (w/v) to about 0.2% (w/v) with respect to the total weight of the composition. More specifically, the ginsenoside or a pharmaceutically acceptable salt thereof may be included in an amount of about 0.01% (w/v) to about 0.2% (w/v), about 0.02% (w/v) to about 0.1% (w/v), about 0.03% (w/v) to about 0.08% (w/v), or about 0.04% (w/v) to about 0.06% (w/v) with respect to the total weight of the composition.

The curcumin or a pharmaceutically acceptable salt thereof and the ginsenoside or a pharmaceutically acceptable salt thereof may be in the form of a mixture, and the mixing ratio of the curcumin or a pharmaceutically acceptable salt thereof and the ginsenoside or a pharmaceutically acceptable salt thereof may be 1:10 to 10:1.

In this case, the ginsenoside may include Rh1, Rh2, Rb, Ro or a mixture of all thereof, but is not limited thereto. Ginsenoside Rh1, Rh2, Rb, and Ro are the same as described above.

The pharmaceutical composition of the present invention may further contain at least one compound selected from the group consisting of sulfasalazine, mesalazine (5-aminosalicytic acid, 5-ASA), sucralfate, pentosan polysulfate (PPS), butyric acid, prednisolone, azathioprine, tofacitinib, ruscogenin, and pentoxifylline.

The sulfasalazine, mesalazine (5-aminosalicytic acid, 5-ASA), sucralfate, pentosan polysulfate (PPS), butyric acid, prednisolone, azathioprine, tofacitinib, ruscogenin and pentoxifylline are as described above.

In one embodiment of the present invention, the pharmaceutical composition may further include sucralfate and/or butyric acid. In this case, the pharmaceutical composition may contain sucralfate or butyric acid in an amount of 0.001 wt% to 20 wt% with respect to the total weight of the composition. Specifically, the sucralfate may be contained in an amount of about 0.02% (w/v) to about 0.5% (w/v) with respect to the total weight of the composition. More specifically, the sucralfate may be contained in an amount of about 0.02% (w/v) to about 0.5% (w/v), about 0.03% (w/v) to about 0.4% (w/v), about 0.04% (w/v) to about 0.3% (w/v), about 0.05% (w/v) to about 0.2% (w/v), or about 0.06% (w/v) to about 0.1% (w/v).

Specifically, the butyric acid may be contained in an amount of about 0.0002% (w/v) to about 0.02% (w/v) with respect to the total weight of the composition. More specifically, the butyric acid may be contained in an amount of about 0.0002% (w/v) to about 0.02% (w/v), about 0.0004% (w/v) to about 0.01% (w/v), about 0.0008% (w/v) to about 0.008% (w/v), or about 0.001% (w/v) to about 0.004% (w/v).

The pharmaceutical composition may include a mixture of curcumin or a pharmaceutically acceptable salt thereof, ginsenoside or a pharmaceutically acceptable salt thereof, and sucralfate. The mixing ratio is as described above.

The pharmaceutical composition may include a mixture of curcumin or a pharmaceutically acceptable salt thereof, ginsenoside or a pharmaceutically acceptable salt thereof, and butyric acid. The mixing ratio is as described above.

The pharmaceutical composition may include a mixture of curcumin or a pharmaceutically acceptable salt thereof, ginsenoside or a pharmaceutically acceptable salt thereof, and butyric acid and sucralfate. The mixing ratio is as described above.

The pharmaceutical composition of the present invention may be used for the purpose of preventing or treating an inflammatory bowel disease.

The term "inflammatory bowel disease" used herein refers to a disease that causes chronic inflammation in the gastrointestinal tract, and may include all inflammatory diseases occurring in the intestines, such as infectious enteritis caused by bacteria, viruses, amoebic, and tuberculous enteritis, ischemic enteropathy, and radiation enteritis.

In particular, the inflammatory bowel disease may be an inflammatory bowel disease caused by radiation exposure. In this case, the radiation exposure may be a side effect of radiation treatment that occurs when even normal tissue is exposed to radiation during radiation treatment for cancer. Radiation therapy for cancer that may cause the inflammatory bowel disease includes, but is not limited to, treatment methods for colorectal cancer, uterine cancer, prostate cancer, colon cancer, and rectal cancer.

The enteritis induced by radiation exposure may be any one selected from the group consisting of radiation proctitis, radiation enteritis, radiation proctosigmoiditis, radiation-induced ulcer, radiation necrosis, and radiation proctocolitis.

In addition, in the present specification, the inflammatory bowel disease may be any one selected from enteritis, ulcerative colitis, Crohn's disease, intestinal Bechet's disease, hemorrhagic rectal ulcer, and ileal pouchitis.

When the pharmaceutical composition for preventing and treating the inflammatory bowel disease is used for an intestinal disease induced by radiation exposure, the pharmaceutical composition may be administered before or after radiation exposure to prevent or treat enteritis induced by radiation exposure. Preferably, it may be administered after radiation exposure in radiation therapy, but is not limited thereto.

As used herein, the term "treatment" may be used to mean both therapeutic and prophylactic treatment. At this time, prophylaxis may be used to mean that a pathological condition or disease of a subject is alleviated or mitigated. At this time, the term "treatment" includes both application or any form of administration for treating a disease in a mammal, including a human. In addition, the term includes inhibiting or slowing down the progression of a disease; and includes meanings of restoring or repairing impaired or lost function so that a disease is partially or completely alleviated; stimulating inefficient processes; or alleviating a serious disease.

Meanwhile, the pharmaceutical composition of the present invention is administered in a therapeutically effective amount.

The term "administration" used herein means introducing a predetermined substance into a subject in an appropriate manner, and the administration route of the composition may be administered through any general route as long as the composition may reach the target tissue. The pharmaceutical composition may be administered orally or parenterally, and may be administered intraperitoneally, intravenously, intramuscularly, subcutaneously, intradermally, topically, intranasally, intrapulmonary, or rectally as a parenteral administration method. Specifically, it may be administered topically or applied topically to the lesion site, but is not limited thereto.

As a specific example of administration, the composition of the present invention may be used for inhalation administration by being contained in an injection device in the form of a syringe or a spray injection device. For example, this may include an injection device in the form of a tube, but is not limited thereto. In addition, in the case of including an injection device in the form of a syringe, after inserting a discharge portion corresponding to a syringe needle into the lesion site of the subject, a discharge pressure application portion corresponding to a piston of a syringe may be operated to administer the composition of the present invention contained in a liquid form to the lesion site. In this case, the shape of the injection device is not limited to the conventional syringe form.

In the case of including an injection device in the form of a spray injection device, as in the case of using an injection device in the form of a syringe, a discharge portion may be positioned at the lesion site, and then an injection pressure may be applied to administer the composition of the present invention in the form of a spray to the lesion site. In the case of including an injection device in the form of a tube, as described above, a discharge portion may be inserted into the lesion site of the subject, and then the composition of the present invention may be injected into the lesion site in the form of a liquid by applying pressure to the tube. The form of the injection device described above is only a specific example, and any device capable of injecting the composition of the present invention into the lesion site may be used without any particular limitation.

As used herein, the term "therapeutically effective amount" or "pharmaceutically effective amount" refers to an amount of a compound or composition effective to prevent or treat the disease in question, which is sufficient to treat the disease at a reasonable benefit/risk ratio applicable to medical treatment and does not cause adverse effects. A level of the effective amount may be determined depending on factors including the patient's health condition, type and severity of disease, activity of drug, the patient's sensitivity to drug, mode of administration, time of administration, route of administration and excretion rate, duration of treatment, combined or simultaneously used drugs, and other factors well known in the medical field. In one embodiment, the therapeutically effective amount means an amount of drug effective to treat a disease.

The preferred dosage of the pharmaceutical composition may be in the range of 0.01 µg/kg to 10 g/kg or 0.01 mg/kg to 1 g/kg per day depending on the patient's condition, weight, sex, age, severity of the patient, and route of administration. Administration may be once or several times divided a day. Such a dosage should not be construed as limiting the scope of the present invention in any way.

The pharmaceutical composition of the present invention may be administered as an individual therapeutic agent or in combination with another therapeutic agent, may be administered sequentially or simultaneously with a conventional therapeutic agent, and may be administered once or multiple times. Considering all of the above factors, it is important to administer an amount that may achieve the maximum effect with the minimum amount without side effects, and this may be easily determined by those skilled in the art.

The pharmaceutical composition of the present invention may be produced in the form of a unit dose by formulating it using a pharmaceutically acceptable carrier and/or excipient according to a method that may be easily performed by a person skilled in the art to which the present invention pertains, or may be produced by placement thereof in a large-capacity container, and may further include a dispersant or stabilizer.

The term "pharmaceutically acceptable carrier" used in the present invention includes any and all solvents, dispersion media, coatings, antibacterial agents, antifungal agents, and isotonic agents. In addition to conventional media or preparations that are immiscible with the active ingredient, its use in therapeutic compositions is contemplated. In addition, supplementary active ingredients may be incorporated into the composition.

When the pharmaceutical composition is produced as a parenteral formulation, preparations for parenteral administration may include sterile aqueous solutions, non-aqueous solvents, suspensions, emulsions, lyophilized preparations, and suppositories. Non-aqueous solvents and suspensions may include propylene glycol, polyethylene glycol, vegetable oils such as olive oil, and injectable esters such as ethyl oleate. As a suppository base, witepsol, macrogol, tween 61, cacao butter, laurin oil, glycerogelatin, etc. may be used. Formulation of pharmaceutical composition is known in the art, and specifically, reference may be made to the literature [Remington's Pharmaceutical Sciences (19th ed., 1995)], etc. The above literature is considered as a part of this specification.

Another aspect of the present invention provides use of a pharmaceutical composition comprising curcumin and ginsenoside as active ingredients for protecting against or alleviating radiation-induced damage.

Another aspect of the present invention provides use of a pharmaceutical composition comprising curcumin and ginsenoside as active ingredients for preventing or treating an inflammatory bowel disease.

The curcumin, the ginsenoside, the pharmaceutical composition, the radiation-induced damage, and the inflammatory bowel disease are the same as described above.

Another aspect of the present invention provides a method for protecting against or alleviating radiation-induced damage, including administering to a subject a pharmaceutical composition comprising curcumin and ginsenoside as active ingredients.

Another aspect of the present invention provides a method for preventing or treating an inflammatory bowel disease, including administering to a subject a pharmaceutical composition comprising curcumin and ginsenoside as active ingredients.

The curcumin, the ginsenoside, the pharmaceutical composition, the radiation-induced damage, and the inflammatory bowel disease are the same as described above.

The "subject" may be a mammal, preferably a human. In addition, the subject may be a patient suffering from a disease or a subject likely to suffer from a disease.

Route of administration, dosage, and frequency of administration of the composition may vary depending on the patient's condition and the presence or absence of side effects, and thus the composition may be administered to a subject in various ways and amounts. The optimal administration method, dosage, and frequency of administration can be selected in an appropriate range by those of ordinary skill in the art. In addition, the composition may be administered in combination with any compound or natural extract known to have an amelioration effect on the diseases, or may be formulated in the form of combination preparations with other drugs.

### Pharmaceutical composition comprising extract containing curcumin and extract containing ginsenoside

Another aspect of the present invention provides a pharmaceutical composition for protecting against or alleviating radiation-induced damage, comprising as active ingredients: a curcuma extract or a turmeric extract; and a ginseng extract or a red ginseng extract.

The curcuma extract and the turmeric extract may include curcumin, and the ginseng extract and the red ginseng extract may include ginsenoside. The curcumin, the ginsenoside, and the radiation-induced damage are the same as described above.

The term "curcuma (Curcuma longa Linne)" used herein refers to a tuberous root that has been steamed and dried without or after removing the pericarp. This is native or cultivated in Southeast Asia including southern China, India, and Okinawa, and is also cultivated in the central and southern regions of Korea. The original name curcuma (wulgeum) was given because this turns golden (geum) yellow when mixed with alcohol, and other names include masul, hwangul, eulgeum, geolgeum, okgeum, wang, and simhwang.

The term "turmeric" used herein refers to a perennial herbaceous plant belonging to the ginger family, native to hot and humid southern Asian regions such as India and China. In India and other places, the root and stem are peeled, cooked, dried, and powdered to be used as food additives, spices, and coloring agents. In addition, it has recently been suggested that this may be used as a raw material for functional foods such as herbal medicine compositions for preventing and treating liver disease. In Korea, turmeric has been used as a folk therapeutic agent effective for choleretic action, diuretic action, and promoting gastric juice secretion. In addition, "curcumin" is the main medicinal ingredient of turmeric, and has been known to have anticancer, antioxidant, anti-inflammatory, detoxifying, and cholesterol-lowering effects in animal experiments and epidemiological studies.

The term "ginseng" used herein is a perennial semi-shade herbaceous plant belonging to the Areliaceae family, Panax genus, and the root thereof is called Ginseng (Ginseng radix) and is used for medicinal purposes. The scientific name is Panax ginseng C.A Meyer, and the etymology of "Panax" is a compound word of the Greek words Pan (all) and Axos (cure), meaning to cure all diseases. According to the "Ginseng Industry Act" of Korea, "ginseng" refers to fresh ginseng, red ginseng, Taegeuk ginseng, white ginseng, and other ginsengs. Ginseng is classified into fresh ginseng that is dug up from the ground and unprocessed, red ginseng that has been steamed and dried according to the processing method, white ginseng that has been cut off the fine roots of fresh ginseng, slightly peeled off the skin, and dried, and Taegeuk ginseng that has been washed with water, soaked in hot water for a certain period of time, and then dried. "Other ginsengs" refer to those produced using fresh ginseng as a raw material (excluding red ginseng, Taegeuk ginseng, and white ginseng) as specified by the Ministry of Agriculture, Food and Rural Affairs. Fresh ginseng contains 70% to 75% or more moisture, making it difficult to store without special storage facilities or packaging.

The term "red ginseng" used herein refers to a herbal medicine prescription made by steaming and drying ginseng. There are various methods of producing red ginseng, and one of them is the method of production using a non-evaporating pot. When making red ginseng, there is a production method called 9Jeung-9Po, which involves repeating steaming and drying nine times, and a production method of steaming and drying using high-pressure steam, etc., has been developed. This is a production method in which the ginseng is steamed and dried for 18 to 24 hours or more in a non-evaporating pot designed to prevent the release of ginseng components, which includes 9 to 18 hours required for steaming by nine times.

In the present invention, the curcuma, turmeric, ginseng and red ginseng extracts may be obtained by extraction and separation from nature. Specifically, those obtained by extraction and separation from various organs of natural, hybrid or mutant plants may be used. For example, they may be extracted from roots, stems, leaves, flowers, fruit bodies, fruit peels, as well as plant tissue cultures. Preferably, they may be extracted from roots.

The term "extract" used herein includes extracts themselves obtained by curcuma, turmeric, ginseng and red ginseng extraction treatment, diluted or concentrated extracts, dried products obtained by drying the extracts, adjusted or purified products of the extracts, or mixtures thereof, as well as extracts in all formulations that may be formed using the extracts. The extracts of the present invention may be produced in the form of a liquid, or a dried powder by an additional process such as freeze drying or spray drying after extraction before use.

In the present invention, the extract may be an extract extracted from curcuma, turmeric, ginseng or red ginseng, respectively. In the present invention, the extract may be an extract extracted from a mixture of curcuma or turmeric and ginseng or red ginseng. In this case, curcuma or turmeric and ginseng or red ginseng may be mixed at a ratio of 1:10 to 10:1. As one embodiment, this may be an extract extracted after mixing curcuma and ginseng at a ratio of 1:10 to 10:1. As one embodiment, this may be an extract extracted after mixing curcuma and red ginseng at a ratio of 1:10 to 10:1. As one embodiment, this may be an extract extracted by mixing turmeric and ginseng at a ratio of 1:10 to 10:1. As one embodiment, this may be an extract extracted by mixing turmeric and red ginseng at a ratio of 1:10 to 10:1.

In the present invention, the extract may be a solvent extract extracted with an extraction solvent, a fraction obtained by adding a fractionation solvent to an extract prepared by extracting with an extraction solvent, or a purified product obtained by chromatography of the fraction, but is not limited thereto. The extraction solvent may be water, an organic solvent, or a mixed solvent thereof that may be used for extracting natural products. The extraction solvent may be various solvents such as purified water, ethanol, methanol, butanol, acetone, chloroform, and ethyl acetate, used alone or in combination. The extract of the present invention may be produced according to a conventional method for producing plant extracts. More specifically, the production of the extract may be performed by adding an extraction solvent to a dried product of curcuma, turmeric, ginseng, and red ginseng from which impurities have been removed, or a pulverized product of the dried product, and extracting the dried product or pulverized product.

The extraction method using the solvent may be a cold immersion extraction method, a hot water extraction method, a solvent extraction method, a reflux cooling extraction method, or an ultrasonic extraction method, and may preferably be a reflux cooling extraction method, but is not limited thereto.

The extract may be obtained by a method in which curcuma, turmeric, ginseng, or red ginseng is incubated in a solvent and an active ingredient is extracted in the solvent. The incubation may be performed at, but is not limited to, a temperature of about 4°C to reflux, about 4°C to about 100°C, about 4°C to about 80°C, about 4°C to about 60°C, about 4°C to about 50°C, about 4°C to about 40°C, about 4°C to about 35°C, about 4°C to about 30°C, about 4°C to about 25°C or room temperature. The incubation may be performed for a sufficient time for the active ingredient to be extracted into the solvent. The time may be 1 hour to 2 weeks, 1 hour to 1 week, 1 hour to 5 days, 1 hour to 3 days, 12 hours to 5 days, 12 hours to 3 days, 12 hours to 2 days, 24 hours to 5 days or 24 hours to 3 days, but is not limited thereto. The extraction may be performed 1 to 7 times, preferably 1 to 4 times, and more preferably 1 to 3 times, but is not limited thereto.

As an example of the present invention, the extract may be cooled and extracted using ethanol or methanol in a reflux cooling extractor at 80°C for 1 hour to 4 hours, and the above process is repeated 3 times to obtain the extract.

In addition, the extract used in the present invention may undergo an additional fractionation process and may be purified using a purification method. For example, additional purified fractions may be obtained using various chromatography such as high performance liquid chromatography, medium pressure liquid chromatography, thin layer chromatography, etc.

There is no limitation on the method for producing the extract used in the present invention and the method may be changed.

Therefore, the extract in the present invention may include any of the extracts, fractions, purified products, dilutions thereof, and concentrated and dried products obtained in all stages of extraction, fractionation, and purification.

The pharmaceutical composition may contain 0.001 wt% to 20 wt% of the curcuma or turmeric extract as an active ingredient with respect to the total weight of the composition. Specifically, the pharmaceutical composition may contain about 0.03% (w/v) to about 0.4% (w/v) of the curcuma or turmeric extract with respect to the total weight of the composition. More specifically, the curcuma or turmeric extract may be contained in an amount of about 0.03% (w/v) to about 0.4% (w/v), about 0.04% (w/v) to about 0.3% (w/v), about 0.05% (w/v) to about 0.2% (w/v), or about 0.06% (w/v) to about 0.1% (w/v).

In addition, the pharmaceutical composition may contain 0.001 wt% to 20 wt% of the ginseng or red ginseng extract as an active ingredient with respect to the total weight of the composition. Specifically, the pharmaceutical composition may contain about 0.03% (w/v) to about 0.4% (w/v) of the ginseng or red ginseng extract with respect to the total weight of the composition. More specifically, the ginseng or red ginseng extract may be contained in an amount of about 0.03% (w/v) to about 0.4% (w/v), about 0.04% (w/v) to about 0.3% (w/v), about 0.05% (w/v) to about 0.2% (w/v), or about 0.06% (w/v) to about 0.1% (w/v).

In the present invention, the pharmaceutical composition may include a mixture of each of the curcuma extract or turmeric extract and the ginseng extract or red ginseng extract. In this case, the mixing ratio thereof may be 1:10 to 10:1. As one embodiment, this may be a mixture of curcuma extract and ginseng extract mixed at a ratio of 1:10 to 10:1. As one embodiment, this may be a mixture of curcuma extract and red ginseng extract mixed at a ratio of 1:10 to 10:1. As one embodiment, this may be a mixture of turmeric extract and ginseng extract mixed at a ratio of 1:10 to 10:1. As one embodiment, this may be a mixture of turmeric extract and red ginseng extract mixed at a ratio of 1:10 to 10:1.

In the present invention, the pharmaceutical composition may include an extract obtained by mixing curcuma or turmeric and ginseng or red ginseng followed by extraction. In this case, the mixing ratio is the same as described above.

The pharmaceutical composition of the present invention is used for protecting tissue exposed to radiation or promoting recovery of damaged areas by suppressing inflammatory reactions in tissue caused by radiation exposure.

Therefore, the pharmaceutical composition may be administered before or after radiation exposure to protect tissue from damage caused by radiation or to cure damaged tissue. Preferably, the pharmaceutical composition may be administered after radiation exposure in radiation therapy, but is not limited thereto.

Another aspect of the present invention provides a pharmaceutical composition for preventing or treating an inflammatory bowel disease, comprising as active ingredients: a curcuma extract or a turmeric extract; and a ginseng extract or a red ginseng extract. In this case, the curcuma extract and the turmeric extract may contain curcumin, and the ginseng extract and the red ginseng extract may contain ginsenoside. The curcuma, the turmeric, the ginseng, the red ginseng, the extract, the curcumin, the ginsenoside, the inflammatory bowel disease, the prevention and treatment are the same as described above.

The pharmaceutical composition may be administered orally or parenterally. A preferred parenteral administration method may be topical administration or topical application to the lesion site, but is not limited thereto. The administration, the parenteral administration and the topical administration are the same as described above.

The preferred dosage of the pharmaceutical composition may be in the range of 0.01 µg/kg to 10 g/kg or 0.01 mg/kg to 1 g/kg per day depending on the patient's condition, weight, sex, age, severity of the patient, and route of administration. Administration may be once or several times divided a day. Such a dosage should not be construed as limiting the scope of the present invention in any way.

The pharmaceutical composition of the present invention may be administered as an individual therapeutic agent or in combination with another therapeutic agent, may be administered sequentially or simultaneously with a conventional therapeutic agent, and may be administered once or multiple times. Considering all of the above factors, it is important to administer an amount that may achieve the maximum effect with the minimum amount without side effects, and this may be easily determined by those skilled in the art.

The pharmaceutical composition of the present invention may be produced in the form of a unit dose by formulating it using a pharmaceutically acceptable carrier and/or excipient according to a method that may be easily performed by a person skilled in the art to which the present invention pertains, or may be produced by placement thereof in a large-capacity container, and may further include a dispersant or stabilizer.

When the pharmaceutical composition is produced as an oral formulation, it may be produced in a solid form such as a tablet, a pill, powders, a granule, a capsule, etc., and such solid preparation is prepared by mixing the composition with at least one excipient, such as starch, calcium carbonate, sucrose, lactose, gelatin, etc. In addition, simple excipients as well as lubricants such as magnesium stearate and talc are also used. Liquid preparations for oral administration include suspensions, solutions, emulsions, and syrups, and commonly used simple diluents such as water and liquid paraffin, as well as various excipients such as wetting agents, sweeteners, flavoring agents, and preservatives may be included. Regarding the formulation of pharmaceutical compositions, it is known in the art, and specifically, reference may be made to the literature [Remington's Pharmaceutical Sciences (19th ed., 1995)], etc. The above literature is considered as a part of this specification.

Another aspect of the present invention provides use of a pharmaceutical composition comprising a curcuma extract or a turmeric extract; and a ginseng extract or a red ginseng extract as active ingredients for protecting against or alleviating radiation-induced damage.

Another aspect of the present invention provides use of a pharmaceutical composition comprising a curcuma extract or a turmeric extract; and a ginseng extract or a red ginseng extract as active ingredients for preventing or treating an inflammatory bowel disease.

The curcuma, the turmeric, the ginseng, the red ginseng, the extract, the pharmaceutical composition, the radiation-induced damage, and the inflammatory bowel disease are the same as described above.

Another aspect of the present invention provides a method for protecting against or alleviating radiation-induced damage, including administering to a subject a pharmaceutical composition comprising a curcuma extract or a turmeric extract and a ginseng extract or a red ginseng extract as active ingredients.

Another aspect of the present invention provides a method for preventing or treating an inflammatory bowel disease, including administering to a subject a pharmaceutical composition comprising a curcuma extract or a turmeric extract and a ginseng extract or a red ginseng extract as active ingredients.

The curcuma, the turmeric, the ginseng, the red ginseng, the extract, the pharmaceutical composition, the radiation-induced damage, the inflammatory bowel disease, the subject, and the administration are the same as described above.

### Pharmaceutical formulation

### Pharmaceutical formulation comprising curcumin and ginsenoside

Another aspect of the present invention provides a pharmaceutical formulation comprising: (a) curcumin having a structure of Chemical Formula 1 or Chemical Formula 2 below or a pharmaceutically acceptable salt thereof; (b) any one ginsenoside selected from the group consisting of Chemical Formula 3, Chemical Formula 4 and Chemical Formula 5 below or a pharmaceutically acceptable salt thereof; and (c) a gelling polymer:

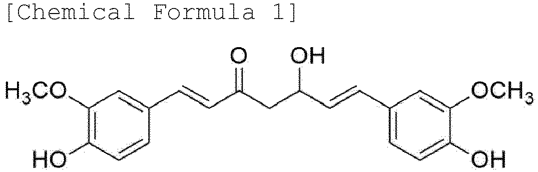

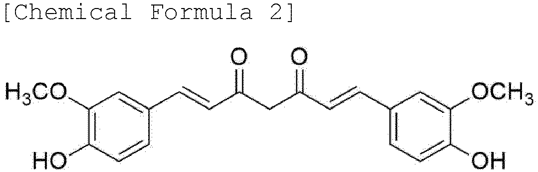

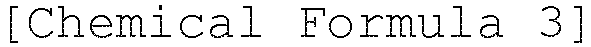

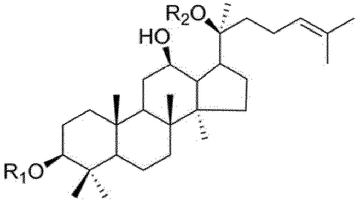

In the formula above, R₁ may be any one selected from the group consisting of H-, Glu- and Glu-Glu-, and R₂ may be any one selected from the group consisting of H-, Glu-, Glu-Glu-, Arap-Glu- and Araf-Glu-.

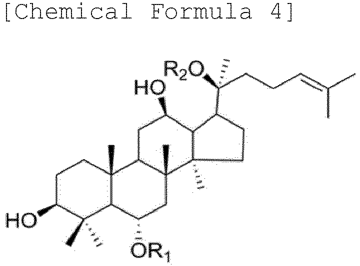

In the formula above, R₁ may be any one selected from the group consisting of H-, Glu-, Rha-Glu- and Xyl-Glu, and R₂ may be H- or Glu-.

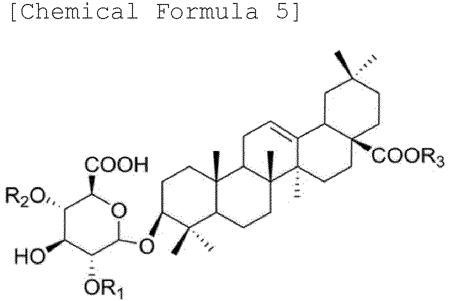

In the formula above, R₁ and R₃ are Glu-, and R₂ is H-.

In Chemical Formulas 3 to 5, Glu is β-D-glucopyranosyl, Arap is α-L-arabinopyranosyl, Araf is α-L-arabinofuranosyl, Xyl is β-D-xylopyranosyl, and Rha is α-L-rhamnopyranosyl.

Here, the curcumin, the ginsenoside and the pharmaceutically acceptable salt are the same as described above.

The pharmaceutical formulation of the present invention may further comprise at least one compound selected from the group consisting of sulfasalazine, mesalazine (5-aminosalicytic acid, 5-ASA), sucralfate, pentosan polysulfate (PPS), butyric acid, prednisolone, azathioprine, tofacitinib, ruscogenin and pentoxifylline.

The sulfasalazine, mesalazine (5-aminosalicytic acid, 5-ASA), sucralfate, pentosan polysulfate (PPS), butyric acid, prednisolone, azathioprine, tofacitinib, ruscogenin, and pentoxifylline are the same as described above.

In the present invention, the curcumin or a pharmaceutically acceptable salt thereof may be included in an amount of about 0.03% (w/v) to about 0.4% (w/v) with respect to the total weight. Specifically, the curcumin or a pharmaceutically acceptable salt thereof may be included in an amount of about 0.03% (w/v) to about 0.4% (w/v), about 0.04% (w/v) to about 0.3% (w/v), about 0.05% (w/v) to about 0.2% (w/v), or about 0.06% (w/v) to about 0.1% (w/v).

The ginsenoside or a pharmaceutically acceptable salt thereof may be included in an amount of about 0.01% (w/v) to about 0.2% (w/v). Specifically, the ginsenoside or a pharmaceutically acceptable salt thereof may be included in an amount of about 0.01% (w/v) to about 0.2% (w/v), about 0.02% (w/v) to about 0.1% (w/v), about 0.03% (w/v) to about 0.08% (w/v), or about 0.04% (w/v) to about 0.06% (w/v).

In this case, the ginsenoside may include Rh1, Rh2, Rb, Ro, or a mixture of all thereof, but is not limited thereto. Ginsenoside Rh1, Rh2, Rb, and Ro are the same as described above.

In addition, as an embodiment of the present invention, the pharmaceutical formulation may further comprise sucralfate and/or butyric acid. Specifically, the pharmaceutical composition may comprise curcumin or a pharmaceutically acceptable salt thereof, a ginsenoside or a pharmaceutically acceptable salt thereof, and sucralfate. The pharmaceutical formulation may comprise a mixture of curcumin or a pharmaceutically acceptable salt thereof, a ginsenoside or a pharmaceutically acceptable salt thereof, and butyric acid. The pharmaceutical composition may comprise a mixture of curcumin or a pharmaceutically acceptable salt thereof, a ginsenoside or a pharmaceutically acceptable salt thereof, butyric acid, and sucralfate.

In this case, the sucralfate may be included in an amount of about 0.02% (w/v) to about 0.5% (w/v). Specifically, the sucralfate may be included in an amount of about 0.02% (w/v) to about 0.5% (w/v), about 0.03% (w/v) to about 0.4% (w/v), about 0.04% (w/v) to about 0.3% (w/v), about 0.05% (w/v) to about 0.2% (w/v), or about 0.06% (w/v) to about 0.1% (w/v).

The butyric acid may be included in an amount of about 0.0002% (w/v) to about 0.02% (w/v). Specifically, the butyric acid may be included in an amount of about 0.0002% (w/v) to about 0.02% (w/v), about 0.0004% (w/v) to about 0.01% (w/v), about 0.0008% (w/v) to about 0.008% (w/v), or about 0.001% (w/v) to about 0.004% (w/v).

The term "gelation" used herein means that when the polymer concentration increases and the dispersed polymer begins to form branches and crosslinks, a sol-gel transition occurs, whereby the sol becomes a gel when a critical concentration is reached. The gel may be considered to be chemically linked or physically linked. Physical gels may in turn be categorized as strong or weak depending on their bonding properties, using strong materials approaching chemical gels in terms of bonding persistence. Strong physical bonding includes lamellar microcrystals, glassy crystals, or double and triple helices, while weak physical gels include hydrogen bonding, block copolymers, micelles, and ionic bonding.

The pharmaceutical formulation of the present invention may be a gel-type formulation that includes one or more gelling polymers. Specifically, the formulation may be a formulation of a water-soluble hydrogel.

The term "hydrogel" as used herein is also called a hydrated gel, and is a hydrophilic polymer network structure that forms a three-dimensional cross-link, has a high water content, and exhibits elasticity almost similar to natural tissue. In addition, this does not dissolve in an aqueous environment and may be made from various polymers, so that this has various chemical compositions and properties. In addition, since this is easy to process, this may be transformed into various forms depending on the application. Hydrogels have high biocompatibility due to high-water content thereof and physicochemical similarity with the extracellular matrix. Hydrogels may exhibit various characteristics depending on the type of the polymer used as the main chain or the crosslinking method adopted.

The pharmaceutical formulation of the present invention may include a mucoadhesive polymer selected from carboxymethylcellulose, carbopol (carbomer, polyacrylic acid polymer), poly(methyl methacrylate), polyacrylamide, polycarbophil, acrylic acid/butyl acrylate copolymer, sodium alginate, and dextran. This may preferably include carbopol, but is not limited thereto. In the present invention, the carbopol may be used as a basic skeleton forming a hydrogel.

Carbopol is a polymer belonging to the carboxyvinyl polymer family, also known as carbomer. The carbopol may be any one selected from carbomers including Carbopol 934, Carbopol 940, Carbopol 950, Carbopol 980, Carbopol 951, and Carbopol 981, but is not limited thereto. In one embodiment of the present invention, the carbopol may be Carbopol 934.

The carbopol may be included in the pharmaceutical formulation of the present invention in an amount of about 0.5% (w/v) to about 2% (w/v). Specifically, this may be included in an amount of about 0.5% (w/v), about 0.6% (w/v), about 0.7% (w/v), about 0.8% (w/v), about 0.9% (w/v), about 1% (w/v), about 1.1% (w/v), about 1.2% (w/v), about 1.3% (w/v), about 1.4% (w/v), about 1.5% (w/v), about 1.6% (w/v), about 1.7% (w/v), about 1.8% (w/v), about 1.9% (w/v) or about 2% (w/v). Preferably, the carbopol may be included in an amount of about 2% (w/v).

In addition, the pharmaceutical formulation may further comprise hyaluronic acid and/or alginate as a gelling polymer.

The "hyaluronic acid" is a type of mucopolysaccharide composed of amino acids and uronic acid, and refers to a high molecular weight compound with a molecular weight of 200,000 to 400,000 in which N-acetylglucosamine and glucuronic acid are alternately bonded in a chain shape, similar to the pectin of plants, and is hydrolyzed by hyaluronidase. In vivo, this exists in the vitreous body of the eye or the umbilical cord, etc., and has a high viscosity and plays a role in preventing bacterial invasion or toxic substance penetration. For the present invention, the hyaluronic acid may be used for the function of delaying the diffusion of drugs in a hydrogel as a basic skeleton forming the hydrogel.

The "alginate" is a natural anionic polysaccharide obtained from brown algae, and has been used for many biomedical applications due to its biocompatibility, low toxicity, low cost, and ability to form a hydrogel by the addition of divalent cations such as Ca²⁺. For the present invention, the alginate may be used for the function of increasing the mucosal adhesion of a hydrogel as a basic skeleton forming the hydrogel.

The hyaluronic acid and/or the alginate may be included in the pharmaceutical formulation of the present invention in an amount of about 0.1% (w/v) to 1% (w/v), respectively. Specifically, this may be included in an amount of about 0.1% (w/v), about 0.2% (w/v), about 0.3% (w/v), about 0.4% (w/v), about 0.5% (w/v), about 0.6% (w/v), about 0.7% (w/v), about 0.8% (w/v), about 0.9% (w/v), or about 1% (w/v). Preferably, the hyaluronic acid may be included at about 0.5% (w/v).

The preservative that may be added to the pharmaceutical formulation may be selected from the group consisting of benzoic acid, sodium benzoate, benzalconic chloride, phenylmercuric nitrate, chlorexidine, paraben, and sorbic acid, but is not limited thereto. Preferably, this may be paraben.

In addition, the pharmaceutical formulation may include a flavoring agent as an additive. The flavoring agent may be, for example, pine oil, lavender oil, peppermint oil, orange oil, lemon oil, eucalyptus oil, and blended flavoring, eucalyptus sting, etc., but is not limited thereto.

The pharmaceutical formulation of the present invention may be administered by a parenteral route. Specifically, the formulation may be administered by a topical route, but is not limited thereto. Preferably, the formulation may be used by a method of topical injection or topical application by direct injection or catheter use to the lesion site. Here, the parenteral, the administration, and the topical administration are the same as described above.

In addition, the pharmaceutical formulation may be used for alleviating or ameliorating radiation-induced damage; or preventing or treating an inflammatory bowel disease. In this case, the radiation-induced damage and the inflammatory bowel disease are the same as described above.

Another aspect of the present invention provides use of the pharmaceutical formulation for the production of a medicament for alleviating or ameliorating radiation-induced damage or for preventing or treating an inflammatory bowel disease. The pharmaceutical formulation, the radiation-induced damage and the inflammatory bowel disease are the same as described above.

### Pharmaceutical formulation comprising extract containing curcumin and extract containing ginsenoside

Another aspect of the present invention provides a pharmaceutical formulation comprising: a curcuma extract or a turmeric extract; a ginseng extract or a red ginseng extract; and a gelling polymer. In this case, the curcuma extract and the turmeric extract may contain curcumin, and the ginseng extract and the red ginseng extract may contain ginsenoside. The curcuma, the turmeric, the ginseng, the red ginseng, the extract, the curcumin, the ginsenoside, and the gelling polymer are the same as described above.

In the present invention, the pharmaceutical formulation may contain the curcuma or the turmeric extract in an amount of about 0.03% (w/v) to about 0.4% (w/v) with respect to the total weight of the preparation. Specifically, the curcuma or the turmeric extract may be contained in an amount of about 0.03% (w/v) to about 0.4% (w/v), about 0.04% (w/v) to about 0.3% (w/v), about 0.05% (w/v) to about 0.2% (w/v), or about 0.06% (w/v) to about 0.1% (w/v).

In addition, the pharmaceutical formulation may contain the ginseng or red ginseng extract in an amount of about 0.03% (w/v) to about 0.4% (w/v), about 0.04% (w/v) to about 0.3% (w/v), about 0.05% (w/v) to about 0.2% (w/v), or about 0.06% (w/v) to about 0.1% (w/v), with respect to the total weight of the preparation.

In the present invention, the pharmaceutical formulation may comprise a mixture of each of the curcuma extract or the turmeric extract and the ginseng extract or the red ginseng extract. In this case, the mixing ratio thereof is the same as described above.

In the present invention, the pharmaceutical formulation may contain an extract obtained by mixing curcuma or turmeric and ginseng or red ginseng followed by extraction. In this case, the mixing ratio is the same as described above.

The pharmaceutical formulation may be used for protecting against or alleviating radiation-induced damage, or preventing or treating an inflammatory bowel disease. The radiation-induced damage and the inflammatory bowel disease are the same as described above.

Another aspect of the present invention provides use of the pharmaceutical formulation for the production of a medicament for protecting against or alleviating radiation-induced damage or preventing or treating an inflammatory bowel disease. The curcuma, the turmeric, the ginseng, the red ginseng, the extract, the pharmaceutical formulation, the radiation-induced damage and the inflammatory bowel disease are the same as described above.

### Health functional food composition

### Health functional food composition comprising curcumin and ginsenoside

Another aspect of the present invention provides a health functional food composition for alleviating or ameliorating radiation-induced damage, comprising as active ingredients: curcumin having a structure of Chemical Formula 1 or Chemical Formula 2 below; and any one ginsenoside selected from the group consisting of Chemical Formula 3, Chemical Formula 4 and Chemical Formula 5 below:

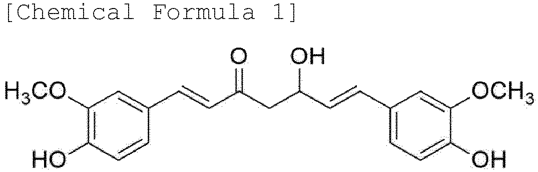

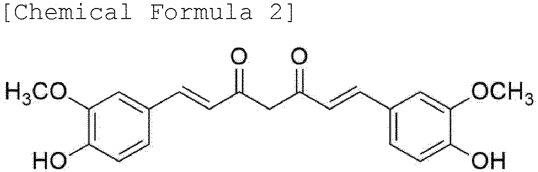

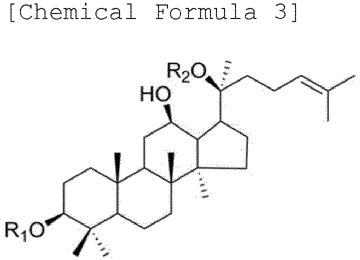

In the formula above, R₁ may be any one selected from the group consisting of H-, Glu- and Glu-Glu-, R₂ may be any one selected from the group consisting of H-, Glu-, Glu-Glu-, Arap-Glu- and Araf-Glu-.

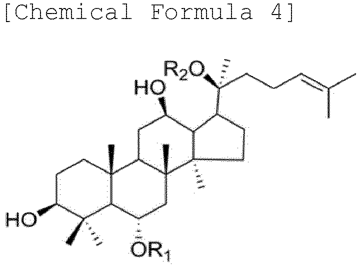

In the formula above, R₁ may be any one selected from the group consisting of H-, Glu-, Rha-Glu- and Xyl-Glu, and R₂ may be H- or Glu-.

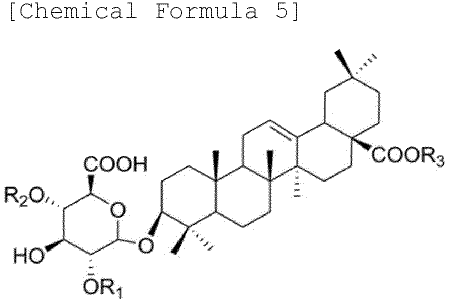

In the formula above, R₁ and R₃ are Glu- and R₂ is H-,

In Chemical Formulas 3 to 5, Glu is β-D-glucopyranosyl, Arap is α-L-arabinopyranosyl, Araf is α-L-arabinofuranosyl, Xyl is β-D-xylopyranosyl, and Rha is α-L-rhamnopyranosyl.

Here, the curcumin, the ginsenoside, the radiation-induced damage, and the alleviating radiation-induced damage are the same as described above.

The term "health functional food" used herein means a food produced and processed using raw materials or ingredients with useful functionality for human body according to Act No. 6727 on Health Functional Foods. "Functionality" here means consumption for regulating nutrients for the structure and function of human body or obtaining useful effects for health purposes such as physiological effects.

The composition according to the present invention may be used as a raw material for a health functional food that alleviates or ameliorates the damage to tissue exposed to radiation by suppressing inflammatory reactions in tissue caused by radiation exposure.

The curcumin, which is an active ingredient, may be contained in an amount of 0.001 wt% to 20 wt% with respect to the total weight of the health food composition. Specifically, the health functional food composition may contain about 0.03% (w/v) to about 0.4% (w/v) of the curcumin with respect to the total weight of the composition. More specifically, the curcumin may be contained in an amount of about 0.03% (w/v) to about 0.4% (w/v), about 0.04% (w/v) to about 0.3% (w/v), about 0.05% (w/v) to about 0.2% (w/v), or about 0.06% (w/v) to about 0.1% (w/v). In addition, the health functional food composition may contain 0.001 wt% to 20 wt% of the ginsenoside, which is an active ingredient, with respect to the total weight of the composition. Specifically, the health functional food composition may contain about 0.01% (w/v) to about 0.2% (w/v) of the ginsenoside with respect to the total weight of the composition. More specifically, the ginsenoside may be contained in an amount of about 0.01% (w/v) to about 0.2% (w/v), about 0.02% (w/v) to about 0.1% (w/v), about 0.03% (w/v) to about 0.08% (w/v), or about 0.04% (w/v) to about 0.06% (w/v).

The curcumin and the ginsenoside may be in the form of a mixture, and the mixing ratio is the same as described above.

In this case, the ginsenoside may include Rh1, Rh2, Rb, Ro, or a mixture of all thereof, but is not limited thereto. Ginsenoside Rh1, Rh2, Rb, and Ro are the same as described above.

The health functional food composition of the present invention may comprise butyric acid, and the butyric acid is the same as described above. The butyric acid may be contained in an amount of about 0.0002% (w/v) to about 0.02% (w/v) with respect to the total weight of the composition. More specifically, the butyric acid may be contained in an amount of about 0.0002% (w/v) to about 0.02% (w/v), about 0.0004% (w/v) to about 0.01% (w/v), about 0.0008% (w/v) to about 0.008% (w/v), or about 0.001% (w/v) to about 0.004% (w/v).

In addition, the composition may comprise a mixture of curcumin, ginsenoside, and butyric acid, and the mixing ratio is the same as described above. However, the composition according to the present invention may be used in amounts below the above range in the case of long-term intake for the purpose of health and hygiene or health control. In addition, it may be used in an amount above the above range in order to obtain the effect of alleviating or ameliorating the damage to the tissue exposed to radiation, so that the range is not limited to the above range.

Another aspect of the present invention provides a health functional food composition for alleviating or ameliorating an inflammatory bowel disease, comprising as active ingredients: curcumin having a structure of Chemical Formula 1 or Chemical Formula 2 below; and any one ginsenoside selected from the group consisting of Chemical Formula 3, Chemical Formula 4 and Chemical Formula 5 below:

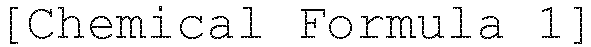

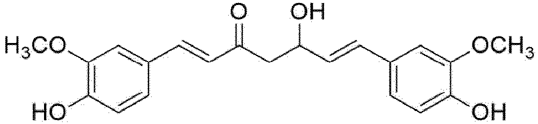

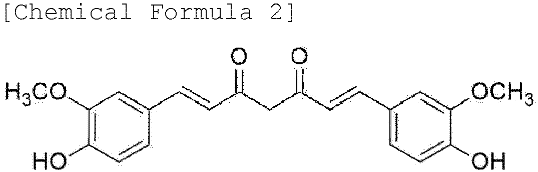

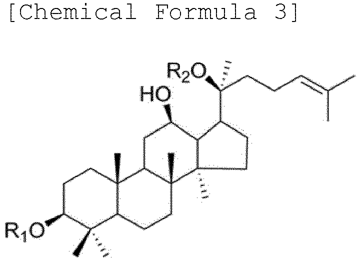

In the formula above, R₁ may be any one selected from the group consisting of H-, Glu- and Glu-Glu-, and R₂ may be any one selected from the group consisting of H-, Glu-, Glu-Glu-, Arap-Glu- and Araf-Glu-.

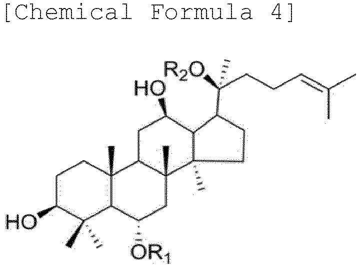

In the formula above, R₁ may be any one selected from the group consisting of H-, Glu-, Rha-Glu- and Xyl-Glu, and R₂ may be H- or Glu-.

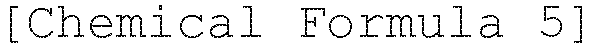

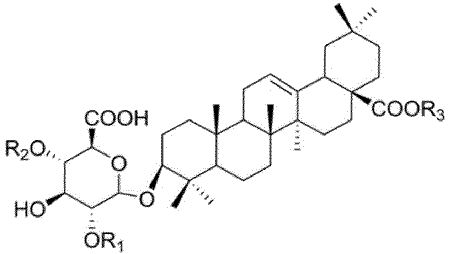

In the formula above, R₁ and R₃ are Glu-, and R₂ is H-.

In Chemical Formulas 3 to 5, Glu is β-D-glucopyranosyl, Arap is α-L-arabinopyranosyl, Araf is α-L-arabinofuranosyl, Xyl is β-D-xylopyranosyl, and Rha is α-L-rhamnopyranosyl.

Here, the curcumin, the ginsenoside, the inflammatory bowel disease, and the health functional food are the same as described above.

The composition according to the present invention may be used as a raw material for a health functional food that alleviates or ameliorates an inflammatory bowel disease.

The curcumin, which is an active ingredient, may be contained in an amount of 0.001 wt% to 20 wt% with respect to the total weight of the health food composition. Specifically, the health functional food composition may contain about 0.03% (w/v) to about 0.4% (w/v) of the curcumin with respect to the total weight of the composition. More specifically, the curcumin may be contained in an amount of about 0.03% (w/v) to about 0.4% (w/v), about 0.04% (w/v) to about 0.3% (w/v), about 0.05% (w/v) to about 0.2% (w/v), or about 0.06% (w/v) to about 0.1% (w/v). In addition, the health functional food composition may contain 0.001 wt% to 20 wt% of the ginsenoside, which is an active ingredient, with respect to the total weight of the composition. Specifically, the health functional food composition may contain about 0.01% (w/v) to about 0.2% (w/v) of the ginsenoside with respect to the total weight of the composition. More specifically, the ginsenoside may be contained in an amount of about 0.01% (w/v) to about 0.2% (w/v), about 0.02% (w/v) to about 0.1% (w/v), about 0.03% (w/v) to about 0.08% (w/v), or about 0.04% (w/v) to about 0.06% (w/v).

The curcumin and the ginsenoside may be in the form of a mixture, and the mixing ratio is the same as described above.

In this case, the ginsenoside may include Rh1, Rh2, Rb, Ro, or a mixture of all thereof, but is not limited thereto. Ginsenoside Rh1, Rh2, Rb, and Ro are the same as described above.

The health functional food composition for alleviating or ameliorating an inflammatory bowel disease of the present invention may comprise butyric acid, and the butyric acid is the same as described above. The butyric acid may be contained in an amount of about 0.0002% (w/v) to about 0.02% (w/v) with respect to the total weight of the composition. More specifically, the butyric acid may be contained in an amount of about 0.0002% (w/v) to about 0.02% (w/v), about 0.0004% (w/v) to about 0.01% (w/v), about 0.0008% (w/v) to about 0.008% (w/v), or about 0.001% (w/v) to about 0.004% (w/v).

Additionally, the composition may comprise a mixture of curcumin, ginsenoside and butyric acid, and the mixing ratio is the same as described above. However, the composition according to the present invention may be used in amounts below the above range in the case of long-term intake for the purpose of health and hygiene or health control. In addition, it may be used in an amount above the above range in order to obtain the effect of alleviating or ameliorating an inflammatory bowel disease, so that the range is not limited to the above range.

The term "ameliorating" used herein means any acts in which the symptoms of a disease are improved or beneficially changed by administration of the composition.

The health functional food composition according to the present invention may be formulated into a formulation of a conventional health functional food known in the art. The health functional food may be produced as, for example, powders, a granule, a tablet, a pill, a capsule, a suspension, an emulsion, a syrup, an infusion, a liquid, an extract, gum, tea, jelly, or beverage. Any carrier or additive known to be usable in the art for the production of the formulation to be produced may be used as the food-wise acceptable carrier or additive. For example, in the case of health functional foods in the form of beverage, preservatives, stabilizers, emulsifiers, dispersants, flavoring agents, coloring agents, etc. included in food additives may be contained as needed. In the case of health functional foods in the form of capsules, hard capsules may be produced by filling a conventional hard capsule with a mixture of curcumin, ginsenoside, and butyric acid and additives such as excipients, or granular or coated granular substances thereof, and soft capsules may be produced by filling a capsule base such as gelatin with a mixture of curcumin, ginsenoside, and butyric acid and additives such as excipients, etc. The soft capsules may contain plasticizers such as glycerin or sorbitol, coloring agents, preservatives, etc. as needed. The health functional food in the form of a pill may be prepared by forming a mixture of curcumin, ginsenoside and butyric acid with excipients, binders, disintegrants, etc. in an appropriate manner, and, as needed, may be coated with white sugar or another appropriate coating agent, or starch, talc or a suitable substance. The health functional food in the form of a granule may be produced into a granule by forming a mixture of curcumin, ginsenoside and butyric acid with excipients, binders, disintegrants, etc. in an appropriate manner, and, as needed, may contain a flavoring agent, a taste-correcting agent, etc. The definitions of the terms for excipients, binders, disintegrants, lubricants, taste-correcting agents, and flavoring agents of the present invention are described in literature known in the art, and include those of which functions are the same or similar (Commentary on the Korean Pharmacopoeia, Moonseongsa, Korean Pharmaceutical College Council, 5th revised edition, pp. 33-48, 1989).

### Health functional food composition comprising extract containing curcumin and extract containing ginsenoside

Another aspect of the present invention provides a health functional food composition for alleviating or ameliorating radiation-induced damage, comprising as active ingredients: a curcuma extract or a turmeric extract; and a ginseng extract or a red ginseng extract.

In addition, another aspect of the present invention provides a health functional food composition for preventing or ameliorating an inflammatory bowel disease, comprising as active ingredients: a curcuma extract or a turmeric extract; and a ginseng extract or a red ginseng extract.

In this case, the curcuma extract and the turmeric extract may contain curcumin, and the ginseng extract and the red ginseng extract may contain ginsenoside. The curcuma, turmeric, ginseng, red ginseng, extract, curcumin, ginsenoside, radiation-induced damage, and inflammatory bowel disease are the same as described above.

In the present invention, the curcuma or turmeric extract, which is an active ingredient, may be contained in an amount of 0.001 wt% to 20 wt% with respect to the total weight of the health food composition. Specifically, the health functional food composition may contain the curcuma or turmeric extract in an amount of about 0.03% (w/v) to about 0.4% (w/v) with respect to the total weight of the composition. More specifically, the curcuma or turmeric extract may be included in an amount of about 0.03% (w/v) to about 0.4% (w/v), about 0.04% (w/v) to about 0.3% (w/v), about 0.05% (w/v) to about 0.2% (w/v), or about 0.06% (w/v) to about 0.1% (w/v).

In addition, in the present invention, the ginseng or red ginseng extract, which is an active ingredient, may be contained in an amount of 0.001 wt% to 20 wt% with respect to the total weight of the health food composition. Specifically, the health functional food composition may contain the ginseng or red ginseng extract in an amount of about 0.03% (w/v) to about 0.4% (w/v) with respect to the total weight of the composition. More specifically, the ginseng or red ginseng extract may be included in an amount of about 0.03% (w/v) to about 0.4% (w/v), about 0.04% (w/v) to about 0.3% (w/v), about 0.05% (w/v) to about 0.2% (w/v), or about 0.06% (w/v) to about 0.1% (w/v).

In the present invention, the health functional food may include a mixture of each of the curcuma extract or the turmeric extract and the ginseng extract or the red ginseng extract. In this case, the mixing ratio thereof is the same as described above.

In the present invention, the health functional food may include an extract obtained by mixing curcuma or turmeric and ginseng or red ginseng followed by extraction. In this case, the mixing ratio is the same as described above.

### MODE FOR CARRYING OUT THE INVENTION

Hereinafter, the present invention will be described in more detail through Examples. These Examples are intended only to illustrate the present invention, and it will be obvious to those skilled in the art that the scope of the present invention is not to be construed as being limited by these Examples.

### I. Efficacy evaluation using formulated effective agent

### Production Example 1. Method for synthesizing formulation comprising effective agents

The types and concentrations of polymers and agents used for synthesizing a formulation are as shown in Table 1.

**[Table 1]**

| Effective agents | Chemical Info. | Concentration |
|---|---|---|
| Curcumin | CAS no. 458-37-7 | 5 mM |
| Sigma Aldrich(Cat# C7727) | M.W. 368.38 g/mol | |
| Ginsenoside, Rh1 | CAS no. 63223-86-9 | 1 mM |
| Sigma Aldrich(Cat# 56805) | M.W. 638.87 g/mol | |
| Butyric acid | CAS no. 107-92-6 | 1 mM |
| Sigma Aldrich(Cat# B103500) | M.W. 88.11 g/mol | |
| Sucralfate | CAS no. 54182-58-0 | 1 mM |
| Cat# Y0001324 | M.W. 2086.75 g/mol | |

| Formulation | Chemical Info. | Concentration |
|---|---|---|
| Carbopol 934P Lubrizol(Cat# CBP1034) | CAS no. 9003-01-04 | 2% (w/v) |
| Hyaluronic acid, Sodium salt | CAS no. 9067-32-7 | 0.5% (w/v) |
| Santa Cruz(Cat# SC-204004C) | | |
| Sodium Alginate | CAS no. 9005-38-3 | 0.5% (w/v) |
| Sigma Aldrich(Cat# PHR1471) | | |

| Additive | Chemical Info. | |
|---|---|---|
| Methylparaben | CAS no. 99-76-3 | 0.16% (w/v) |
| Sanfu Chemical | | 160 mg / 100 g |
| Propylparaben | CAS no. 94-13-3 | 0.04%(w/v) |
| Sanfu Chemical | | 40 mg/100 g |
| Fragrance Mint fragrance(Cat# 20863) | N/A(for food) Ministry of Food and Drug Safety M071843 | 0.5%(w/w) |
| DMSO | CAS no. 67-68-5 | 5% (v/v) |
| Sigma Aldrich | | |

The synthesis of the formulation was carried out by producing a stock through separate hydration of a hydrogel-based main polymer (carbopol) and auxiliary polymers (hyaluronic acid and sodium alginate) that enhance moisture retention, mucoadhesion, and drug delivery rate, and then synthesizing a final formulation base. The procedure is as follows and was prepared with respect to a volume of the final formulation base of 10 ml. Therefore, the final formulation base of 10 ml contained carbopol, hyaluronic acid, sodium alginate, as well as effective agents and additives (preservatives, flavoring agents, etc.). Carbopol 934P was synthesized at a concentration of 2% (w/v) as the main polymer of this formulation. After weighing and aliquoting 0.2 g of carbopol into a 50 mL falcon conical tube, 3 ml of DW was added, and vortexed. As the particle size of the polymer was large, it did not dissolve 100% in the above process. Therefore, the tube was fixed on a rocker and mixing was performed overnight at 4°C. The hydrated polymer was sealed and stored at 4°C, and was consumed within 5 days after synthesis.

Since hyaluronic acid (HA) has a low proportion in the final formulation and is difficult to hydrate in the state of all polymer mixed due to its large particle size, in the present invention, 40 ml of a hyaluronic acid stock with a concentration of 2.5% (w/v, 1 g of hyaluronic acid/40 ml of DW)) was separately prepared and hydrated, and then was used for the synthesis of the final formulation. Specifically, 1 g of hyaluronic acid was weighed in a 50 ml falcon conical tube, 20 ml of DW was added, and the mixture was thoroughly mixed by vortexing. An additional 20 ml of DW was added, the mixture was thoroughly mixed, then the tube was fixed on a rocker and mixing was performed overnight at 4°C. The hyaluronic acid stock prepared by the above method was added at 2 ml per 10 ml of the mixed final formulation to provide a final concentration of 0.5% (w/v).

A sodium alginate (SA) stock was synthesized at a concentration of 1 g/40 ml and prepared to account for 0.5% (w/v) of the final concentration. Since SA is a natural polymer derived from seaweed and is prone to denaturation, a preservative was added to the SA stock during preparation thereof. Specifically, 1 g of SA was weighed in a 50 ml falcon tube, and 320 mg of methylparaben and 80 mg of propylparaben were each sufficiently dissolved in 40 ml of DW. In this case, since solubility in DW is low, some crystals remain undissolved, but the remainders are absorbed into the polymer during the hydration process.

Accordingly, in order to check the degree of dissolution of the materials, the final formulation was applied to a slide glass and examined under a microscope. The quantified SA was added to the solution dissolved by the above method, vortexed, and then 20 ml of the above solution was further added and mixed by inverting. The tube containing the mixture was fixed to a rocker and then mixed overnight at 4°C. Finally, in producing 10 ml of the mixed final formulation, 2 ml of 2 ml of the SA stock was added such that the mixture has a final concentration of 0.5% (w/v).

In order to produce a final formulation base, each stock (3 ml of Carbopol, 2 ml of HA, 2 ml of SA) prepared by the above-described method was quantified and divided into 50 ml conical tubes, and then the effective agents dissolved in DMSO was added and mixed with a disposable pipette. Since the formulation in this state exhibited acidic properties, neutralization was performed using 1 N NaOH. In this case, since the chemical properties of the agents and the formulation may change due to a rapid change in pH when all 1 N of NaOH is added at once, 200 to 500 µl were added at a time and the pH change was checked with a pH stick while adding. In the present invention, approximately 2.5 ml of 1 N NaOH was finally added to perform neutralization. When the pH reached neutral, 250 ul of a flavoring agent was added and mixed. The final combined formulation was a translucent gel-type ointment formulation with a neutral pH and a reddish tint.

### Experimental Example 1. Cell culture

Human colon cancer cell lines (HCT116 and SNU-C1), a human dermal fibroblast cell line (HDF), and a human keratinocyte cell line (HaCaT) were cultured in Dulbecco's modified eagles medium-high glucose (DMEM) containing 10% fetal bovine serum (FBS), 100 units/mL penicillin, and 100 ug/mL streptomycin. A Human umbilical vascular endothelial cell line (HUVEC) was cultured in a vascular cell basal medium (ATCC) containing Endothelial cell growth kit-VEGF (ATCC). All of the above cells were cultured in a 5% CO₂ cell incubator at 37°C.

### Experimental Example 2. Cell viability measurement

Cell viability test was performed using Cell Counting Kit-8. HCT116 and SNU-C1 cell lines, which are colon cancer cells, were seeded on 60 mm culture dishes and cultured, and then irradiated with 10 Gy of radiation. The cells were harvested, seeded on 96-well culture plates, and incubated, and then the cells were treated with butyric acid (BA), ginsenosides Rh1, Rh2, Ro, Rb1, and curcumin (CU) at concentrations of 5, 10, or 50 µM, respectively. After 48 hours, cell viability was analyzed using Cell Counting Kit-8 and a microplate reader.

### Experimental Example 3. Cell mobility measurement

Cell mobility was measured using a 24 mm transwell with 0.4 µm pores. After stabilizing the cells irradiated with 10 Gy of radiation for 24 hours, the cells were suspended in a medium and seeded in the upper chamber of the transwell, and the lower chamber was filled with a medium containing 10% fetal bovine serum (FBS) and 10 µM butyric acid (BA), ginsenosides Rh1, Rh2, Ro, Rb1, curcumin (CU), and sucralfate (SCF) either alone or in combination, and culturing was performed. After 24 hours of culture, the downside of the transwell was stained with crystal violet dye, and the stained cells were counted by observing them with an inverted microscope.

### Experimental Example 4. Examination of vascular endothelial cell function

### Experimental Example 4.1. Examination of vascular endothelial cell tube formation

After irradiating human umbilical endothelial cells (HUVEC) with 10 Gy of radiation, the cells were seeded on a matrigel-coated µ-slide, and were treated with 10 µM of curcumin (CU), butyric acid (BA), sucralfate (SCF), ginsenosides Rh1, Rh2, Rb1, or Ro either alone or in combination. After culturing for 3 hours, randomly selected fields were observed with an inverted microscope to measure the number and length of formed tubes.

### Experimental Example 4.2. Measurement of vascular endothelial growth factor expression

The amount of protein expression of a vascular endothelial growth factor (VEGF) was measured using an enzyme-linked immunosorbent assay (ELISA). After culturing human keratinocytes (HaCaT) treated with 10 µM of curcumin (CU), butyric acid (BA), sucralfate (SCF), ginsenoside Rh1, Rh2, Rb1, or Ro either alone or in combination, the amount of VEGF protein in the culture medium was measured using a microplate reader (Biorad) following the test method provided by the manufacturer using the Human VEGF Quantikine ELISA kit (R&D systems).

### Experimental Example 5. Examination of effect of combined use of effective agents

### Experimental Example 5.1. Cell mobility experiment

The experiment was conducted using the same manner as Experimental Example 3, except that the effective agents for treatment were a combination of curcumin, butyric acid, sucralfate, and ginsenoside.

### Experimental Example 5.2. Endothelial cell function test

The experiment was conducted in the same manner as Experimental Example 4, except that the effective agents for treatment were a combination of curcumin, butyric acid, sucralfate, and ginsenoside.

### Experimental Example 5.3. Examination of changes in inflammatory cytokine expression

Human monocyte cells (THP-1) were differentiated into macrophages using PMA (phorbol 12-myristate 13-acetate). Thereafter, they were treated with lipopolysaccharide (LPS) to induce an inflammatory response, and then treated with a mixture of curcumin (CU), butyric acid (BA), and sucralfate (SCF), and ginsenoside Rh1 in combination. The cell culture was harvested and the expression level of inflammatory cytokines was measured using Luminex Discovery Assay (Human Premixed Multi-Analyte kit, R&D system).

### Experimental Example 6. Statistical analysis

Quantitative data are expressed as mean ± standard error of the mean (SEM). Significant differences relative to each condition were analyzed by one-way analysis of variance (ANOVA) (Tukey's multiple comparison test) using GraphPad PRISM. A p value less than 0.05 was considered to be statistically significant.

### Example 1. Examination of effects of curcumin, butyric acid and ginsenosides on cell viability reduced by irradiation

In order to investigate the effects of curcumin (CU), butyric acid (BA), and ginsenoside on the decreased cell viability due to irradiation, HCT116 and SNU-C1 cells were treated with curcumin, butyric acid, ginsenoside Rh1, Rh2, Ro, or Rb1 at concentrations of 5, 10, and 50 µM, respectively, and the viability of each cell was measured (FIGS. 1 and 2).

As a result, the cell viability of HCT116 and SNU-C1 cells treated with each compound was observed similarly (HCT116 (FIG. 1) and SNU-C1 (FIG. 2)). In each cell, the cell viability decreased by about 49% due to irradiation, and treatment with curcumin (CU) at 5 µM or 10 µM significantly increased the cell viability which had been decreased by irradiation. In the case of the butyric acid (BA) or ginsenoside treatment group, the cell viability was similar to or slightly increased compared to the irradiated group. However, the ginsenoside Rh2 at 50 µM, Ro at 5 µM, and curcumin (CU) at 50 µM treatment groups showed lower cell viabilities than the irradiated group. Through the above results, it was confirmed that curcumin (CU), butyric acid (BA), or ginsenoside 10 µM was the optimal concentration capable of increasing the cell viability decreased by irradiation. Therefore, for the experiments below, all compounds for treatment were at a concentration of 10 µM.

### Example 2. Examination of effects of curcumin, butyric acid and ginsenoside on cell mobility reduced by irradiation

Cellular mobility is one of the fundamental factors that determine the behavior and fate of a cell. It is known that radiation seriously impairs cell mobility, which is essential for damage repair (Partridge 2008). Therefore, the effects of curcumin (CU), ginsenosides (Rh1, Rh2, Ro, and Rb1), and butyric acid (BA) on cell mobility damaged by irradiation were investigated.

As a result, HCT116 cells showed a decrease in the cell mobility rate by approximately 75% due to irradiation (FIG. 3). Cells treated with butyric acid (BA), ginsenoside, or curcumin (CU) each showed a double increase in the cell mobility rate compared to the irradiated group, and among them, the ginsenoside Rh2 treatment group showed the highest cell mobility recovery rate. In SNU-C1 cells, the cell mobility rate decreased by approximately 50% due to irradiation, and showed a tendency for the cell mobility rate to recover by compound treatment (FIG. 4). In particular, ginsenoside Rb1 or curcumin (CU) significantly increased cell mobility compared to the irradiated group. Therefore, through the above results, it was confirmed that butyric acid (BA), ginsenoside, or curcumin may restore cell mobility compromised by irradiation.

### Example 3. Examination of effects of combined treatment with curcumin, butyric acid, sucralfate, and ginsenoside on cell mobility reduced by irradiation

It was investigated whether combined treatment with curcumin (CU), butyric acid (BA), and ginsenoside, including sucralfate (SCF), which has mucosal protective effects as a candidate for a therapeutic agent of proctitis, showed a synergistic recovery effect on the mobility of cells damaged by irradiation.

As a result, treatment with each compound alone increased the cell mobility rate by 28% on average compared to the irradiated group (FIGS. 3 and 4), whereas treatment with the compounds in combination increased the cell mobility rate by approximately 34% (FIG. 5). In particular, when ginsenoside Rh1 or Rb1 was combined with curcumin (CU), butyric acid (BA), and sucralfate (SCF) in HCT116 and SNU-C1 cells, respectively, the cell mobility rate was the highest, and this was higher than that of the group in which all ginsenosides were combined with curcumin (CU), butyric acid (BA), and sucralfate (SCF).

In addition, in order to investigate the effect of combined treatment with the above compounds on the mobility of normal cells instead of cancer cells, the cell mobility rate was determined after combined treatment with the above compounds in human dermal fibroblasts (HDFs) and human keratinocytes (HaCaT). As a result, the cell mobility rate increased by up to about 80% in the combined treatment group compared to the irradiated group (FIG. 6). In particular, in HDF cells, the highest cell mobility recovery rate was observed in the combined treatment group with Rh1 and the combined treatment group with all ginsenosides. In the case of HaCaT cells, the highest cell mobility was observed in the combined treatment group with Rh1, which was similar to the level of cells that had not been irradiated. Therefore, through the above results, it was found that the four compounds were effective in combination in recovering cell mobility induced by irradiation.

### Example 4. Examination of effects of combined treatment with curcumin, butyric acid, ginsenoside, and sucralfate on endothelial cell damage induced by irradiation

The endothelium plays an important role in inflammatory responses, including leukocyte mobilization and cytokine secretion, and is also a radiation-sensitive tissue (Pober and Sessa 2007, Ai, Ye et al. 2020) . This means that damage to the endothelium due to radiation exposure may inhibit the recovery response to radiation. In fact, when HUVEC cells (human umbilical cord vascular endothelial cell line) were irradiated, it was confirmed that tube formation was inhibited (FIG. 7). Therefore, it was investigated whether a combined treatment with curcumin (CU), butyric acid (BA) , sucralfate (SCF), and ginsenoside could restore the function of HUVEC cells damaged by irradiation.

As a result, when irradiated HUVEC cells were treated with the compounds in combination, the number and length of tubes formed increased compared to the irradiated group. In this case, no significant difference was observed between the four ginsenoside treatment groups (FIG. 7 and FIG. 8). In addition, as a result of examining vascular endothelial growth factor (VEGF) (Gupta, Jaskowiak et al. 2002) involved in endothelial cell survival, it was confirmed that the concentration of VEGF decreased by about 50% due to irradiation, but the combined treatment with the compounds increased the concentration of VEGF by about 2 times compared to the irradiated group (FIG. 9). Through the above results, it was confirmed that combined treatment with curcumin (CU), butyric acid (BA), sucralfate (SCF), and ginsenoside could alleviate endothelial damage caused by irradiation.

### Example 5. Examination of effects of curcumin, butyric acid, ginsenoside Rh1 and sucralfate on cell mobility reduced by irradiation

It was investigated whether a treatment with curcumin (CU), butyric acid (BA), ginsenoside Rh1 (Rh1), and sucralfate (SCF) alone or in combination in HDF cells showed a synergistic recovery effect on the mobility of cells damaged by irradiation.

As a result, it was observed that the recovery efficacy in cell mobility reduced by irradiation was the most excellent in the CD+Rh1+SCF+BA, CD+Rh1, or CD+SCF+Rh1 treatment groups compared to the irradiated group (FIG. 10 and Table 2).

**[Table 2]**

| | Average value | | | | | | | | | | | | |
|---|---|---|---|---|---|---|---|---|---|---|---|---|---|
| Treatmen t group | Non-IR | IR | CU+Rh1 +SCF+B A | CU | Rh1 | SCF | BA | CU+Rh 1 | SCF+Rh 1 | BA+Rh 1 | CU+SCF +Rh1 | CU+BA +Rh1 | SCF+BA +Rh1 |
| Mobility rate (%) | 162 | 41 | 140 | 130 | 99 | 91 | 76 | 130 | 87 | 86 | 128 | 129 | 100 |

### Example 6. Examination of effects of curcumin, butyric acid, ginsenoside Rh1, and sucralfate on endothelial cell damage induced by irradiation

It was investigated whether a treatment with curcumin (CU), butyric acid (BA), sucralfate (SCF), and ginsenoside Rh1 (Rh1) alone or in combination restored the function of HUVEC cells damaged by radiation.

As a result, it was observed that the recovery efficacy for tube-forming ability reduced by radiation was the most excellent in the CU+Rh1+SCF+BA, CU+Rh1, or CU+SCF+Rh1 treatment groups compared to the irradiated group (FIG. 11 and Table 3).

**[Table 3]**

| | Average value | | | | | | | | | | | | |
|---|---|---|---|---|---|---|---|---|---|---|---|---|---|
| Treatmen t group | Non-IR | IR | CU+Rh1 +SCF+B A | CU | Rh1 | SCF | BA | CU+Rh 1 | SCF+Rh 1 | BA+Rh 1 | CU+SCF +Rh1 | CU+BA +Rh1 | SCF+BA +Rh1 |
| Tube length (%) | 19 | 11 | 18 | 15 | 15 | 14 | 14 | 17 | 13 | 14 | 16 | 14 | 14 |

### Example 7. Examination of effects of combined treatment with curcumin, butyric acid, ginsenoside Rh1 and sucralfate on inflammatory response induced by irradiation

To investigate the anti-inflammatory activity of a combined use of curcumin (CU), butyric acid (BA), sucralfate (SCF), and ginsenoside Rh1 (Rh1), the expression of inflammatory cytokines was examined through real-time PCR after treatment of THP-1 cells (human monocytic cells), in which an inflammatory response had been induced by lipopolysaccharide (LPS), with the above compounds.

As a result, it was confirmed that the expression of inflammatory cytokines increased by LPS treatment was significantly reduced by CU+BA+Rh1+SCF treatment (FIG. 12).

### Example 8. Examination of effects of curcumin, butyric acid, ginsenoside, and sucralfate in model mouse of radiation proctitis

### Example 8.1. Examination of anti-inflammatory effect by administration of combination drug to proctitis model mouse

C57BL/6 mice (5 weeks old, female) were irradiated with 27 Gy of radiation, and then divided into test groups as shown in Table 4 and FIG. 13. From the next day, samples prepared at concentrations as shown in Table 5 and FIG. 14 (formulated using the method of Example 1) were administered intrarectally at 2-3 day intervals for a total of 6 times (FIG. 16).

**[Table 4]**

| Control | | | Test | | |
|---|---|---|---|---|---|
| Name | Inj. | IR | Name | Inj. | IR |
| Non-IR | - | - | CU+Rh1 | curcumin+Rh1 | O |
| IR alone | - | O | CU+Rh1+SCF | curcumin+Rh1+sucralfate | O |
| Saline | Saline | O | SCF | sucralfate | O |
| Vehicle | Vehicle | O | CU+Rh1+SCF+B A | curcumin+Rh1+sucralfate+butyric acid | O |

**[Table 5]**

| Name | Drug | Conc. | Mass(%) |
|---|---|---|---|
| CU+Rh1 | curcumin | 5 mM | 0.184 |
| | Rh1 | 1 mM | 0.064 |
| CU+Rh1+SCF | curcumin | 5 mM | 0.184 |
| | Rh1 | 1 mM | 0.064 |
| | sucralfate | 1 mM | 0.21 |
| SCF | sucralfate | 1 mM | 0.21 |
| CU+Rh1+SCF+BA | curcumin | 5 mM | 0.184 |
| | Rh1 | 1 mM | 0.064 |
| | sucralfate | 1 mM | 0.21 |
| | butyric acid | 1 mM | 0.008811 |

The efficacy of the treated samples was confirmed 14 days after the final radiation. In this case, except for the non-irradiated group (n=5), all test treatment groups were conducted with 7 mice per group. A flexible plastic tube was inserted into the rectum for administration, and a formulation containing approximately 50 µl of the compound was administered per mouse at a time. The vehicle refers to a formulation that does not contain curcumin (CU), ginsenoside Rh1, butyric acid (BA), and sucralfate (SCF).

After the experiment, the mice were sacrificed, and the intestinal tissue was extracted. The length of the intestine was measured and the pathological morphology of the intestinal and rectal tissues was analyzed, and the expression level of inflammatory cytokine genes in the tissues was examined to investigate the effects of combined administration of curcumin (CU), butyric acid (BA), ginsenoside, and sucralfate (SCF).

As a result, as shown in FIG. 18, it was observed that the intestinal length of the mice decreased due to irradiation compared to the non-irradiated group (non-IR). However, no difference was observed between the irradiated group (IR-Vehicle) and the sample treatment group.

### Example 8.2. Analysis of pathological morphology of rectal tissue

In addition, pathological morphological analysis of the intestinal tissue was performed by observing the morphology of each treatment group through a microscope after performing H&E staining.

Specifically, the extracted organs were fixed in 10% formalin and paraffin blocks were created (STP120 Spin tissue Processor, Myr). Thereafter, sections were mounted on slides using a microtome (Finesse ME Microtome, Thermo Shandon), dried, deparaffinized and hydrated to create tissue slides. The slides were stained with hematoxylin for 10 minutes, washed, and then stained with eosin for 2 minutes to complete the H&E staining. Hematoxylin stained the nuclei of cells in the tissue, and eosin stained the cytoplasm of cells. In this case, the nuclei of cells in the tissue were stained purple, and the cytoplasm was stained pink.

As a result, compared to the non-irradiated group (non-IR), epithelium loss and crypt damage were observed overall in the saline treatment group (IR+saline) and the vehicle treatment group (IR+Vehicle). However, in the case of the sample treatment group, it was observed that the epithelium loss and crypt damage induced by irradiation were recovered in all sample treatment groups except the sucralfate treatment group (IR+SCF) compared to the irradiated group (IR) (FIGS. 19 to 22).

### Example 8.3. Examination of inflammatory cytokine gene expression in rectal tissue

The gene expression level of inflammatory cytokines in the intestinal tissue was confirmed using the following method. Frozen colon tissue was made into a powder form and dissolved in TRIzol to extract RNA. The extracted RNA was used as a template to synthesize cDNA using RTase, and this was amplified by real-time PCR to analyze the gene expression level of inflammatory cytokines.

As a result, it was confirmed that the gene expression level of most inflammatory cytokines decreased in all sample treatment groups compared to the irradiated group (IR) (FIG. 23). In addition, although the inflammation-alleviating effect was observed in the vehicle treatment group (IR+Vehicle), no effect was not observed in the above pathological morphology analysis, so that it is thought that the inflammation-alleviating effect by vehicle treatment is not significant.

### Example 8.4. Examination of apoptosis and cell proliferation in rectal tissue

### Example 8.4.1. Examination of apoptosis in rectal tissue

In order to examine the degree of apoptosis in intestinal tissue, TUNEL staining was performed, and the morphology of each treatment group was observed with a microscope.

Specifically, the tissue section samples obtained from each treatment group were treated with proteinase K (Dako), reacted for 10 minutes, and then washed with PBS for 4 minutes. The washing process was repeated twice. In this case, proteinase K was prepared by dissolving in 10 mM Tris/HCl (pH 7.4-8.0) at 21~37°C for 15-30 minutes to make 20 ug/ml. The section was treated with 3% H₂O₂ and reacted at room temperature for 10 minutes to block the activity of endogenous peroxidase. The process of washing with PBS for 5 minutes was repeated 3 times.

Then, TUNEL staining was performed using the ApopTag^{®} Plus Peroxidase In Situ Apoptosis Detection Kit (Merk, S7101) as follows. After incubation for 1 minute at room temperature with equilibration buffer, TdTenzyme was treated and reacted at 37°C for 1 hour. The sample was washed for 10 minutes using Stop/Wash Buffer, treated with anti-digoxigenin-peroxidase antibody, and reacted at room temperature for 30 minutes. After the reaction was completed, treatment with a DAB solution afforded color development. The color-developed sample was counterstained with Mayer's Hematoxylin for 2 minutes, sealed with Crystal Mount, and the degree of apoptosis in the rectal tissue was observed with a fluorescence microscope.

As a result, as shown in FIG. 24, it was confirmed that apoptosis was reduced in the sample treatment group compared to the irradiated group (IR alone).

### Example 8.4.2. Examination of cell proliferation in rectal tissue

In order to examine the degree of cell proliferation in rectal tissue, the expression of proliferating cell nuclear antigen (PCNA) in the rectal tissue was determined through immunohistochemistry.

Specifically, tissue section samples were prepared in the same manner as in Example 8.4.1 above, and then treated with anti-PCNA antibody (Transduction laboratories, 610664) diluted 1:200 and reacted at 37°C for 1 hour. The samples were treated with UltraTek anti-biotinylated polyvalent-UltraTek HRP antibody (Scytek, AMG080) as a secondary antibody and reacted for 10 minutes. After the reaction was completed, washing was performed with PBS and treatment with DAB afforded color development. The color-developed sample was counterstained with Mayer's Hematoxylin for 2 minutes, sealed with Crystal Mount, and the degree of PCNA expression in the rectal tissue was observed with a microscope.

As a result, as shown in FIGS. 25 and 26, it was confirmed that cell proliferation (brown area) increased in the sample treatment group compared to the irradiated group (IR alone).

### Example 8.4.3. Analysis of the ratio of apoptosis and cell proliferation

The area of the stained area was quantified using the methods in Example 8.4.1 and Example 8.4.2, and the ratio of apoptosis and cell proliferation was analyzed. The image quantification was performed using Image J, and 3 to 6 observed images were randomly selected for each treatment group.

As a result, as shown in FIG. 27, it was confirmed that the ratio of apoptosis and cell proliferation was lower in the sample treatment group compared to the irradiated group (IR alone). The results indicate that apoptosis is low and cell proliferation is high.

### Example 8.5. Examination of infiltration of immune cells in rectal tissue

### Example 8.5.1. Examination of T cell infiltration in rectal tissue

To examine the degree of T cell infiltration in the intestinal tissue, the expression level of CD4 in the intestinal tissue was determined through immunohistochemistry.

For immunohistochemistry, LSAB 2 kit (DAKO, K0675) was used, and 0.01 M PBS (pH 7.2) was used as a washing buffer. An anti-CD4 antibody (Cell signaling, 25229) was used as the primary antibody at a dilution of 1:100. A tissue section sample prepared was treated with 3% H₂O₂, reacted for 5 minutes, and then washed with a washing buffer for 10 minutes. Then, the sample was treated with the primary antibody, reacted overnight at 4°C, and then washed with a buffer for 10 minutes. The sample was treated with a secondary antibody (antibody included in the LSAB 2 kit), reacted at room temperature for 30 minutes, washed, and then treated with a DAB solution. After reacting at room temperature for 3 minutes, counterstaining was performed with Meyer's Hematoxylin for 0.5 to 1 minute. The results were observed with a microscope.

As a result, as shown in FIG. 28, it was confirmed that the infiltration of CD4 positive cells (T cells) was reduced in the sample treatment group compared to the irradiated group (IR alone). Through the results, it was possible to confirm the anti-inflammatory activity of the sample against the inflammatory response induced by irradiation.

In addition, the area where CD4 was expressed was quantified and presented as a graph (FIG. 29). As a result, as in the results of FIG. 28, it was confirmed that the overall trend of CD4 positive cell (T cell) infiltration was reduced in the sample treatment group compared to the irradiated group (IR alone), and it was confirmed that the phenomenon of reduced T cell infiltration was most prominent in the group administered with sucralfate (SCF), curcumin (CU), and ginsenoside Rh1 (Rh1) in combination.

### Example 8.5.2. Examination of macrophage infiltration in rectal tissue

In order to examine the degree of macrophage infiltration in intestinal tissue, the degree of CD68 expression in the intestinal tissue was determined through immunohistochemistry.

It was performed in the same manner as Example 8.5.1, and the primary antibody was an anti-CD68 antibody (cell signaling, 97778) diluted 1:1000. In addition, the CD68 expression area in the observed image (FIG. 30) was quantified and presented as a graph in FIG. 31.

As a result, as shown in FIG. 30 and FIG. 31, it was confirmed that the infiltration of CD68 positive cells (monocytes) was reduced in the sample treatment group compared to the irradiated group (IR alone). Through the results, it was possible to confirm the anti-inflammatory activity of the sample against the inflammatory response induced by irradiation.

### Example 8.6. RIS evaluation in rectal tissue

In order to evaluate the radiation injury score (RIS) in rectal tissue, each parameter was evaluated by quantification based on the criteria shown in FIG. 32. In this case, the higher the digitized RIS score is, the higher the damage to the rectum due to radiation is.

As a result, as shown in FIG. 33, it was confirmed that RIS value decreased in overall in the sample treatment group compared to the irradiated group (B, score 1.965), and it was confirmed that the value significantly decreased in the group (D) co-administered with curcumin (CU) and ginsenoside (Rh1).

### II. Efficacy evaluation using ginseng extract and turmeric extract

### Production Example 2. Production of extract containing curcumin

### Production Example 2.1. Production of curcuma extract

The curcuma (Jindo Agricultural Cooperative, Haenam Curcuma Farm) used as the raw material was washed cleanly with distilled water, and dried at 80°C using a hot air dryer. After drying, this was pulverized with a grinder, ethanol (v/w) equivalent to 10 times the weight of curcuma was added, and cooling extraction was performed in a reflux cooling extractor at 80°C for 2 hours. After cooling, the supernatant was collected, and the remaining residue was extracted again for 1 hour with the same solvent equivalent to 5 times the weight of curcuma. The above process was repeated twice, and the supernatant was collected. After mixing all the supernatants collected during the extraction process, the mixture was concentrated under reduced pressure at 37°C using a rotary evaporator (IKA RV 10), and filtered using a 0.45 um membrane filter. HPLC was performed with the above extract to confirm that 235 mg/kg of curcumin was contained in the extract. The extract was stored frozen at -20°C before use in the experiment.

### Production Example 2.2. Production of turmeric extract

Turmeric (Jindo Turmeric Agricultural Cooperative) was extracted in the same manner as Production Example 2.1, and HPLC was used to confirm that the extract contained 294.2 mg/kg of curcumin. The extract was stored frozen at -20°C before use in the experiment.

### Production Example 3. Production of ginsenoside-containing extract

### Production Example 3.1. Production of ginseng extract

Ginseng (4-year-old ginseng grown in a test field of KT&G Central Raw Materials Research Institute) was freeze-dried as a whole root without separating the fine roots and rootlets, and then ground in a grinder. An organic solvent (v/w) of 50% ethanol equivalent to 10 times the weight of ginseng was added, and cooling extraction was performed for 2 hours in a reflux cooling extractor at 80°C. After cooling, the supernatant was collected, and the remaining residue was extracted again for 1 hour with the same solvent equivalent to 5 times the weight of ginseng. The above process was repeated twice and the supernatant was collected. After mixing all the supernatants collected during the extraction process, they were concentrated under reduced pressure at 37°C using a rotary evaporator (IKA RV 10). After concentration, the residue was dissolved in 20% acetonitrile, pretreated for solid phase extraction (SPE) using a Sep-Pak C18 cartridge (Waters, Milford), and filtered using a 0.45 um membrane filter. HPLC was performed with the above extract to confirm that 67.44 mg/g of ginsenoside was contained in the extract. The extract was stored frozen at -20°C before use in the experiment.

### Production Example 3.2. Production of red ginseng extract

Red ginseng (4-year-old red ginseng root that meets the standard specifications for residual pesticides, main metals, etc. from Cheonji Agricultural Cooperative) was freeze-dried without separating the main body, fine roots, and rootlets, and then a red ginseng extract was prepared in the same manner as Production Example 2.4, and HPLC was performed to confirm that 33.9 mg/100 ml of ginsenoside was contained in the extract. The extract was stored frozen at - 20°C before use in the experiment.

### Production Example 4. Production of mixture of curcumin-containing extract and ginsenoside-containing extract

A turmeric extract and a ginseng extract extracted in the same manner as Production Examples 2 and 3 were mixed as shown in Table 6 below to prepare an extract mixture containing curcumin and ginsenoside.

**[Table 6]**

| Ginsenoside-containing extract | | Curcumin-containing extract | |
|---|---|---|---|
| Extract | Concentration(mg/ml) | Extract | Concentration(mg/ml) |
| ginseng | 32 | turmeric | 74 |

### Example 9. Examination of effects of mixture of ginseng extract and turmeric extract in model mouse of radiation proctitis

A mixture of ginseng and turmeric extracts was administered orally or rectally to model mice of radiation proctitis established in the same manner as Example 8.1. In this case, ginseng and turmeric extracts were mixed and used in a ratio of 3:7.

Six mice were administered per test group, and the administration concentrations are described in Table 7 below. For a single administration, 100 ul of the extract was administered per mouse for a total of 14 days. During the administration period, general symptoms and visual observations were performed, and body weights were measured. After the end of administration, the mice were sacrificed, intestinal tissue was extracted, and the efficacy of the extract was evaluated through pathological morphological analysis of the rectal tissue.

**[Table 7]**

| Control group | | | Test group | | |
|---|---|---|---|---|---|
| Treatment | Inj. | IR | Administration method | Concentration µg) | IR |
| Non-irradiated group | - | - | Oral administration | 30 | O |
| | | | | 100 | O |
| | | | | 300 | O |
| IR irradiated group | - | O | Rectal administration | 30 | O |
| | | | | 100 | O |
| | | | | 300 | O |

### Example 9.1. Observation of pathological morphology of rectal tissue

In the same manner as Example 8.2, H&E staining was performed on the rectal tissue among the excised intestinal tissue, and the pathological morphology of the rectal tissue was observed.

As a result, as shown in FIG. 34, compared to the non-irradiated group (normal, non-IR), the irradiated group (disease animal model, IR alone) showed overall epithelial loss and crypt damage. On the other hand, in the test group, the ginseng and turmeric extract mixture-administration group, it was confirmed that the epithelial loss and crypt damage were recovered overall compared to the irradiated group (IR alone) .

### Example 9.2. Examination of cell proliferation within rectal tissue

By confirming the expression of PCNA in rectal tissue in the same manner as Example 8.4.2, cell proliferation within rectal tissue was evaluated.

As a result, as shown in FIG. 35, it was confirmed that cell proliferation within the crypt was reduced overall in the irradiated group (IR alone) compared to the non-irradiated group (non-IR). On the other hand, it was confirmed that cell proliferation was enhanced in the extract mixture-administration group.

## Claims

1. A pharmaceutical composition for protecting against or alleviating radiation-induced damage, comprising as active ingredients:
curcumin having a structure of Chemical Formula 1 or Chemical Formula 2 below or a pharmaceutically acceptable salt thereof; and
any one ginsenoside selected from the group consisting of Chemical Formula 3, Chemical Formula 4 and Chemical Formula 5 below or a pharmaceutically acceptable salt thereof: 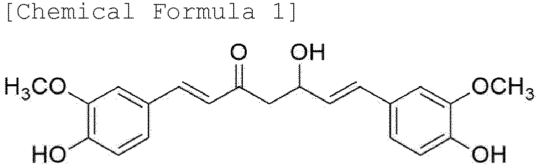 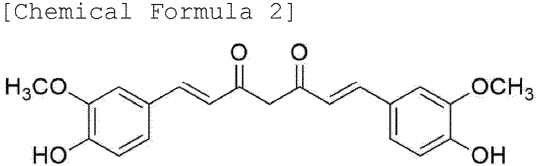 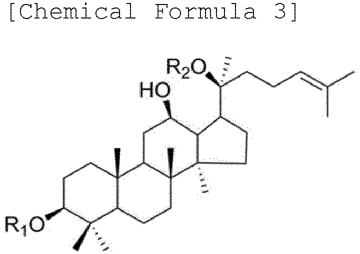 wherein, in the formula above,
R₁ is H-, Glu- or Glu-Glu-, and
R₂ is H-, Glu-, Glu-Glu-, Arap-Glu- or Araf-Glu-, 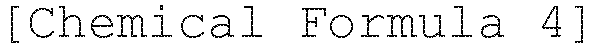 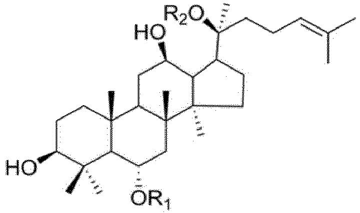 wherein, in the formula above,
R₁ is H-, Glu-, Rha-Glu- or Xyl-Glu, and R₂ is H- or Glu-, 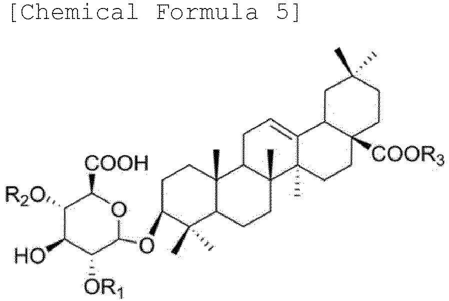
wherein, in the formula above, R₁ and R₃ are Glu-, and R₂ is H-,
wherein the Glu is β-D-glucopyranosyl, Arap is α-L-arabinopyranosyl, Araf is α-L-arabinofuranosyl, Xyl is β-D-xylopyranosyl, and Rha is α-L-rhamnopyranosyl.

2. The pharmaceutical composition for protecting against or alleviating radiation-induced damage of claim 1, wherein the pharmaceutical composition further comprises at least one compound selected from the group consisting of sulfasalazine, mesalazine (5-aminosalicytic acid, 5-ASA), sucralfate, pentosan polysulfate (PPS), butyric acid, prednisolone, azathioprine, tofacitinib, ruscogenin, and pentoxifylline.

3. The pharmaceutical composition for protecting against or alleviating radiation-induced damage of claim 1, wherein the radiation-induced damage is vascular damage, tissue damage, tissue inflammation or tissue fibrosis due to radiation exposure.

4. The pharmaceutical composition for protecting against or alleviating radiation-induced damage of claim 1, wherein the pharmaceutical composition is administered before or after radiation exposure.

5. The pharmaceutical composition for protecting against or alleviating radiation-induced damage of claim 4, wherein the pharmaceutical composition is administered after radiation exposure during radiation therapy.

6. A pharmaceutical composition for preventing or treating an inflammatory bowel disease, comprising active ingredients:
curcumin having a structure of Chemical Formula 1 or Chemical Formula 2 below or a pharmaceutically acceptable salt thereof; and
any one ginsenoside selected from the group consisting of Chemical Formula 3, Chemical Formula 4 and Chemical Formula 5 below or a pharmaceutically acceptable salt thereof: 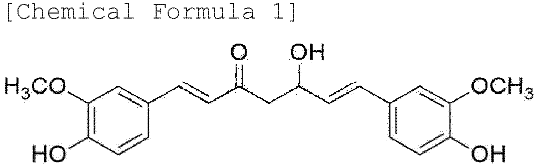 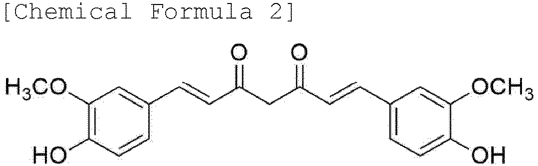 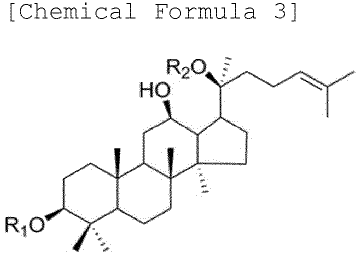 wherein, in the formula above,
R₁ is H-, Glu- or Glu-Glu-, and
R₂ is H-, Glu-, Glu-Glu-, Arap-Glu- or Araf-Glu-, 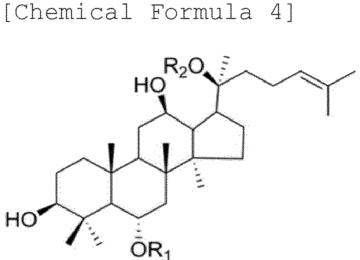 wherein, in the formula above,
R₁ is H-, Glu-, Rha-Glu- or Xyl-Glu, and
R₂ is H- or Glu-, 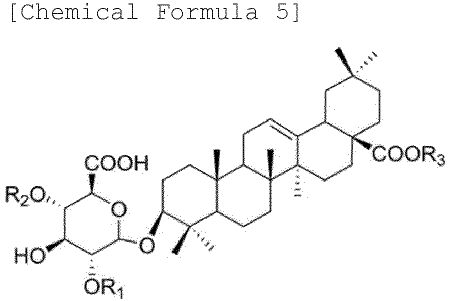
where, in the formula above, R₁ and R₃ are Glu- and R₂ is H-,
wherein the Glu is β-D-glucopyranosyl, Arap is α-L-arabinopyranosyl, Araf is α-L-arabinofuranosyl, Xyl is β-D-xylopyranosyl, and Rha is α-L-rhamnopyranosyl.

7. The pharmaceutical composition for preventing or treating an inflammatory bowel disease of claim 6, wherein the pharmaceutical composition further comprises at least one compound selected from the group consisting of sulfasalazine, mesalazine (5-aminosalicytic acid, 5-ASA), sucralfate, pentosan polysulfate (PPS), butyric acid, prednisolone, azathioprine, tofacitinib, ruscogenin, and pentoxifylline.

8. The pharmaceutical composition for preventing or treating an inflammatory bowel disease of claim 6, wherein the inflammatory bowel disease is an inflammatory bowel disease caused by radiation exposure.

9. The pharmaceutical composition for preventing or treating an inflammatory bowel disease of claim 8, wherein the inflammatory bowel disease is at least one selected from the group consisting of radiation proctitis, radiation enteritis, radiation proctosigmoiditis, radiation-induced ulcer, radiation necrosis, and radiation proctocolitis.

10. The pharmaceutical composition for preventing or treating an inflammatory bowel disease of claim 6, wherein the inflammatory bowel disease is at least one selected from enteritis, ulcerative colitis, Crohn's disease, intestinal Bechet's disease, hemorrhagic rectal ulcer, and ileal pouchitis.

11. The pharmaceutical composition for preventing or treating an inflammatory bowel disease of claim 6, wherein the pharmaceutical composition is administered before or after radiation exposure.

12. The pharmaceutical composition for preventing or treating an inflammatory bowel disease of claim 11, wherein the pharmaceutical composition is administered after radiation exposure in radiation therapy.

13. A pharmaceutical formulation comprising:
(a) curcumin having a structure of Chemical Formula 1 or Chemical Formula 2 below or a pharmaceutically acceptable salt thereof;
(b) any one ginsenoside selected from the group consisting of Chemical Formula 3, Chemical Formula 4 and Chemical Formula 5 below or a pharmaceutically acceptable salt thereof; and
(c) a gelling polymer: 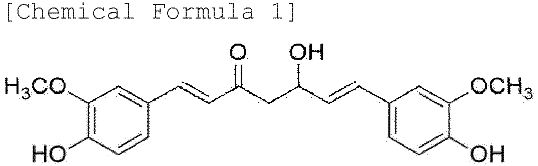 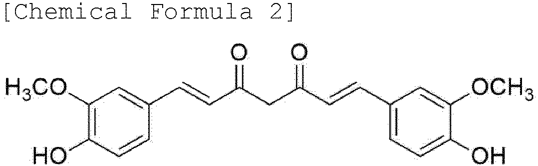 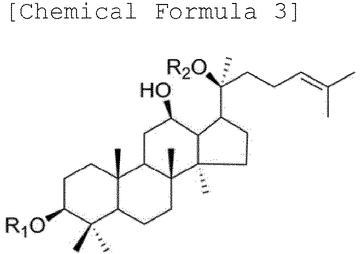 wherein, in the formula above,
R₁ is H-, Glu- or Glu-Glu-, and
R₂ is H-, Glu-, Glu-Glu-, Arap-Glu- or Araf-Glu-, 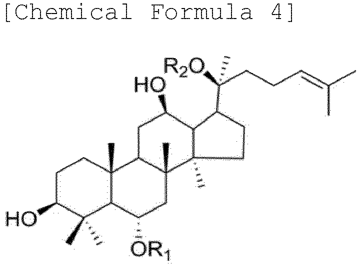 wherein, in the formula above,
R₁ is H-, Glu-, Rha-Glu- or Xyl-Glu, and
R₂ is H- or Glu-, 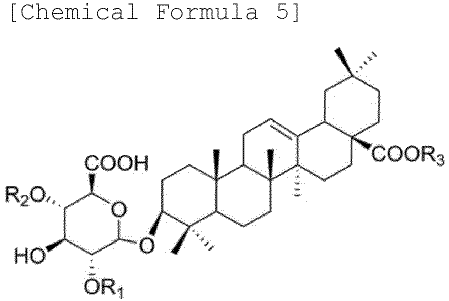
wherein, in the formula above, R₁ and R₃ are Glu-, and R₂ is H-,
wherein the Glu is β-D-glucopyranosyl, Arap is α-L-arabinopyranosyl, Araf is α-L-arabinofuranosyl, Xyl is β-D-xylopyranosyl, and Rha is α-L-rhamnopyranosyl.

14. The pharmaceutical formulation of claim 13, wherein the curcumin or the pharmaceutically acceptable salt thereof is contained in an amount of 0.03% (w/v) to 0.4% (w/v) .

15. The pharmaceutical formulation of claim 13, wherein the ginsenoside or the pharmaceutically acceptable salt thereof is contained in an amount of 0.01% (w/v) to 0.2% (w/v) .

16. The pharmaceutical formulation of claim 13, wherein the pharmaceutical composition further comprises at least one compound selected from the group consisting of sulfasalazine, mesalazine (5-aminosalicytic acid, 5-ASA), sucralfate, pentosan polysulfate (PPS), butyric acid, prednisolone, azathioprine, tofacitinib, ruscogenin, and pentoxifylline.

17. The pharmaceutical formulation of claim 13, wherein the formulation further comprises a preservative or a flavoring agent.

18. The pharmaceutical formulation of claim 13, wherein the formulation is a water-soluble hydrogel.

19. The pharmaceutical formulation of claim 13, wherein the gelling polymer comprises carbopol.

20. The pharmaceutical formulation of claim 19, wherein the carbopol is contained in a concentration of 0.5% (w/v) to 2% (w/v).

21. The pharmaceutical formulation of claim 20, wherein the carbopol is contained in a concentration of 2% (w/v) .

22. The pharmaceutical formulation of claim 13, wherein the gelling polymer further comprises hyaluronic acid and/or alginate.

23. The pharmaceutical formulation of claim 22, wherein the hyaluronic acid is contained in a concentration of 0.1% (w/v) to 1% (w/v).

24. The pharmaceutical formulation of claim 23, wherein the hyaluronic acid is contained in a concentration of 0.5% (w/v).

25. The pharmaceutical formulation of claim 22, wherein the alginate is contained in a concentration of 0.1 to 1% (w/v).

26. The pharmaceutical formulation of claim 25, wherein the alginate is contained in a concentration of 0.5% (w/v) .

27. The pharmaceutical formulation of claim 13, wherein the formulation is administered by a parenteral route.

28. The pharmaceutical formulation of claim 27, wherein the formulation is administered by a topical route.

29. The pharmaceutical formulation of claim 13, wherein the formulation is for the prevention or treatment of an inflammatory bowel disease.

30. A health functional food composition for alleviating or ameliorating radiation-induced damage, comprising as active ingredients:
curcumin having a structure of Chemical Formula 1 or Chemical Formula 2 below; and
any one ginsenoside selected from the group consisting of Chemical Formula 3, Chemical Formula 4 and Chemical Formula 5 below: 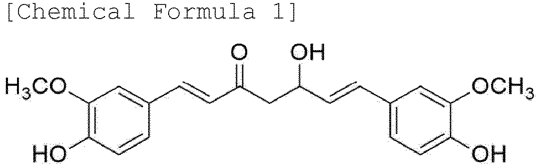 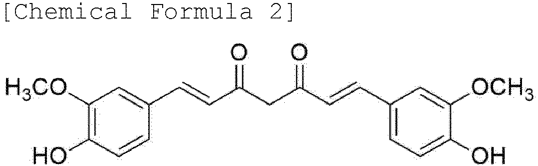 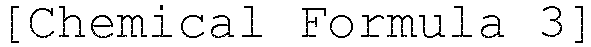 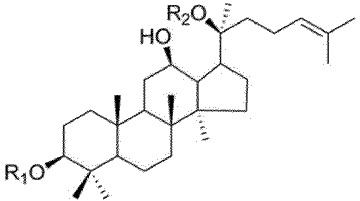 wherein, in the formula above,
R₁ is H-, Glu- or Glu-Glu-, and
R₂ is H-, Glu-, Glu-Glu-, Arap-Glu- or Araf-Glu-, 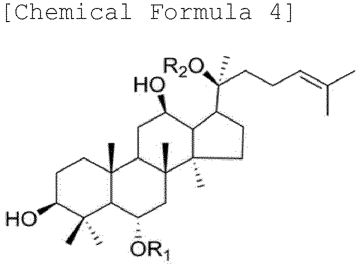 wherein, in the formula above,
R₁ is H-, Glu-, Rha-Glu- or Xyl-Glu, and
R₂ is H- or Glu-, 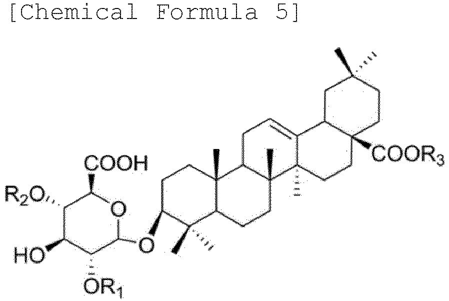
wherein, in the formula above, R₁ and R₃ are Glu-, and R₂ is H-,
wherein the Glu is β-D-glucopyranosyl, Arap is α-L-arabinopyranosyl, Araf is α-L-arabinofuranosyl, Xyl is β-D-xylopyranosyl, and Rha is α-L-rhamnopyranosyl.

31. The health functional food composition for alleviating or ameliorating radiation-induced damage of claim 30, wherein the health functional food composition further comprises butyric acid.

32. A health functional food composition for alleviating or ameliorating an inflammatory bowel disease, comprising as active ingredients:
curcumin having a structure of Chemical Formula 1 or Chemical Formula 2 below; and
any one ginsenoside selected from the group consisting of Chemical Formula 3, Chemical Formula 4 and Chemical Formula 5 below: 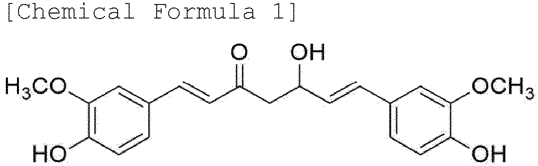 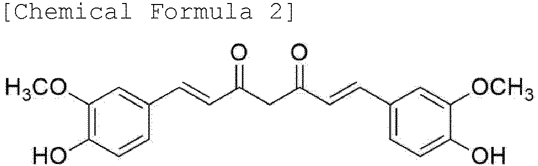 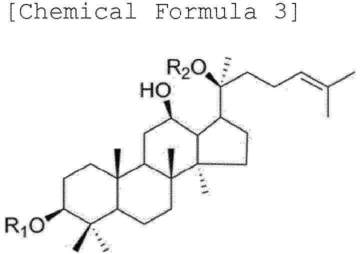 wherein, the formula above,
R₁ is H-, Glu- or Glu-Glu-, and
R₂ is H-, Glu-, Glu-Glu-, Arap-Glu- or Araf-Glu-, 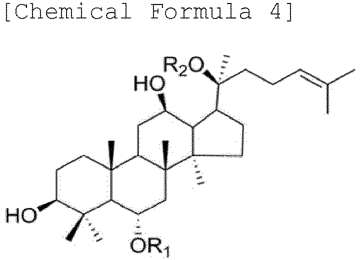 wherein, in the formula above,
R₁ is H-, Glu-, Rha-Glu- or Xyl-Glu, and
R₂ is H- or Glu-, 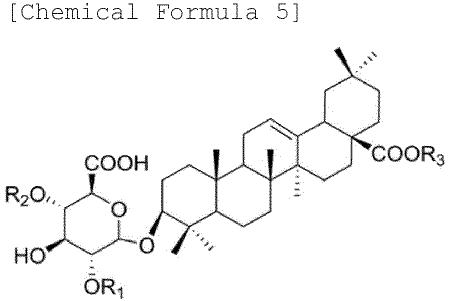
wherein, in the formula above, R₁ and R₃ are Glu- and R₂ is H-,
wherein the Glu is β-D-glucopyranosyl, Arap is α-L-arabinopyranosyl, Araf is α-L-arabinofuranosyl, Xyl is β-D-xylopyranosyl, and Rha is α-L-rhamnopyranosyl.

33. The health functional food composition for alleviating or ameliorating an inflammatory bowel disease of claim 32, wherein the health functional food composition further comprises butyric acid.

34. A pharmaceutical composition for protecting against or alleviating radiation-induced damage, comprising as active ingredients:
a curcuma extract or a turmeric extract; and
a ginseng extract or a red ginseng extract.

35. A pharmaceutical composition for preventing or treating an inflammatory bowel disease, comprising as active ingredients:
a curcuma extract or a turmeric extract; and
a ginseng extract or a red ginseng extract.

36. The pharmaceutical composition for preventing or treating an inflammatory bowel disease of claim 35, wherein the inflammatory bowel disease is at least one selected from the group consisting of radiation proctitis, radiation enteritis, radiation proctosigmoiditis, radiation-induced ulcer, radiation necrosis, and radiation proctocolitis.

37. The pharmaceutical composition for preventing or treating an inflammatory bowel disease of claim 35, wherein the inflammatory bowel disease is at least one selected from enteritis, ulcerative colitis, Crohn's disease, intestinal Bechet's disease, hemorrhagic rectal ulcer, and ileal pouchitis.

38. A health functional food composition for alleviating or ameliorating radiation-induced damage, comprising as active ingredients:
a curcuma extract or a turmeric extract; and
a ginseng extract or a red ginseng extract.

39. A health functional food composition for preventing or ameliorating an inflammatory bowel disease, comprising as active ingredients:
a curcuma extract or a turmeric extract; and
a ginseng extract or a red ginseng extract.

40. Use of the pharmaceutical composition of claim 1 or 34 for protecting against or alleviating radiation-induced damage.

41. Use of the pharmaceutical composition of claim 6 or 35 for preventing or treating an inflammatory bowel disease.

42. A method for protecting against or alleviating radiation-induced damage, including administering to a subject the pharmaceutical composition of claim 1 or 34.

43. A method for preventing or treating an inflammatory bowel disease, including administering to a subject the pharmaceutical composition of claim 6 or 35.

44. Use of the pharmaceutical formulation of claim 13 for the production of a medicament for alleviating or ameliorating radiation-induced damage or for preventing or treating an inflammatory bowel disease.
